# EUROPEAN PATENT SPECIFICATION

(11) **EP 3 075 353 B1**
(45) Date of publication and mention of the grant of the patent: **12.02.2020**
(21) Application number: 16162653.6
(22) Date of filing: 29.03.2016
(51) Int. Cl.: A61F 2/16

(54) **INTRAOCULAR LENS INJECTION INSTRUMENT**
EINSPRITZINSTRUMENTE FÜR INTRAOKULARLINSE
INSTRUMENT D'INJECTION DE LENTILLE INTRAOCULAIRE

(30) Priority: 30.03.2015 JP 2015069149; 30.03.2015 JP 2015069156; 02.03.2016 JP 2016040327
(43) Date of publication of application: 05.10.2016
(60) Divisional application: 19190360.8
(73) Proprietor: NIDEK CO., LTD, Gamagori-shi Aichi 443-0038 (JP)
(72) Inventor: NATSUME, Akiyoshi, Aichi, 443-0038 (JP); NAGASAKA, Shinji, Aichi, 443-0038 (JP); INOUE, Takanori, Aichi, 443-0038 (JP)
(74) Representative: Prüfer & Partner mbB Patentanwälte · Rechtsanwälte

(56) References cited:
- EP-A1- 2 168 535
- US-A1- 2012 221 102
- US-A1- 2014 135 783
- US-A1- 2014 194 890

## Description

### BACKGROUND OF THE INVENTION

### Field of the Invention

The present disclosure relates to an intraocular lens injection instrument for injecting an intraocular lens into a patient's eye.

### Related Art

Heretofore, as one method for cataract surgery, a method of injecting a foldable soft intraocular lens (IOL) into an eye as a substitute for a crystalline lens has been typically used. Further, there is another case that an IOL is injected anterior to the lens for the purpose of correcting a refractive power of an eye. For injection of an IOL into an eye, an intraocular lens injection instrument called as an injector is used in some cases.

One example of an injector has been known with a configuration that an injection part formed with a cutout at its leading end includes a hollow portion having an inner diameter gradually decreasing toward the leading end, and an IOL is pushed out in the hollow portion and folded into a tiny piece so as to be injected outside from the leading end (for example, see Patent Document JP 2010-082288 A).

Further, US 2014/194890 A1 discloses an intraocular lens injection device according to the preamble of claim 1.

US 2014/135783 A1 discloses a hingeless cartridge for intraocular lens injector in which a lumen is tilted relative to plunger axis.

US 2012/221102 A1 discloses a intraocular lens insertion device having a interfering action section which curves and deforms a support section of an intraocular lens in a direction in which the support section approaches an optical section of the intraocular lens.

### SUMMARY OF INVENTION

### Problems to be Solved by the Invention

In the above mentioned injector, a support part on a leading end side of the hollow portion is folded and stored to fold an IOL, but there is a case that the folded optical part fails to store the support part on the leading end side when the IOL is injected into an eye.

The present disclosure has been made to address the above problem and has a purpose to provide an intraocular lens injection instrument configured to appropriately deform and inject an IOL.

### Means of Solving the Problems

The above mentioned purpose is achieved by the intraocular lens injection instrument according to claim 1.

### Effects of the Invention

According to an intraocular lens injection instrument of the present disclosure, an IOL can be appropriately deformed and injected.

### BRIEF DESCRIPTION OF THE DRAWINGS

FIG. 1 is a plan view of an intraocular lens injection instrument;
FIG. 2 is a side view of the intraocular lens injection instrument;
FIG. 3 is a cross-sectional view taken along a line A-A in FIG. 2 in a first example of a first embodiment;
FIG. 4 is a cross-sectional view taken along a line B-B in FIG. 2 in the first example of the first embodiment;
FIG. 5 is a cross-sectional view taken along a line C-C in FIG. 4;
FIG. 6 is a plan view of an intraocular lens (IOL);
FIG. 7 is a right-side view of the IOL;
FIG. 8 is a sectional view showing a state in which a distal end portion of a front-side support part enters inside an outer circumferential part of an optical part;
FIG. 9 is a sectional view showing a state in which the IOL is rotated in a circumferential direction of the optical part in the first example of the first embodiment;
FIG. 10 is a cross-sectional view taken along a line D-D in FIG. 9;
FIG. 11 is a sectional view showing a state in which the IOL is folded into a tiny piece;
FIG. 12 is a sectional view showing a modified example of the first example of the first embodiment;
FIG. 13 is a sectional view showing a comparative example;
FIG. 14 is a cross-sectional view taken along the line A-A in FIG. 2 in a second example of the first embodiment;
FIG. 15 is a sectional view showing a state in which the IOL is rotated in the circumferential direction of the optical part in the second example of the first embodiment;
FIG. 16 is a sectional view showing a modified example of the second example of the first embodiment;
FIG. 17 is a cross-sectional view taken along the line A-A in FIG. 2 in the second embodiment;
FIG. 18 is a cross-sectional view taken along the line B-B in FIG. 2 in the second embodiment;
FIG. 19 is a cross-sectional view taken along a line E-E in FIG. 18;
FIG. 20 is a sectional view showing a state in which the optical part of the IOL contacts an inside surface of a first protrusion;
FIG. 21 is a cross-sectional view taken along a line F-F in FIG. 20;
FIG. 22 is a sectional view showing a state in which a root portion of a rear-side support part of the IOL contacts an inside surface of a second protrusion;
FIG. 23 is a cross-sectional view taken along a line G-G in FIG. 22;
FIG. 24 is a sectional view showing a state in which the optical part of the IOL is started to be valley-folded to make the IOL folded;
FIG. 25 is a sectional view showing a state in which the IOL is pushed out further from the state shown in FIG. 24; and
FIG. 26 is a sectional view showing a state in which the IOL is folded into a tiny piece.

### DESCRIPTION OF EMBODIMENTS

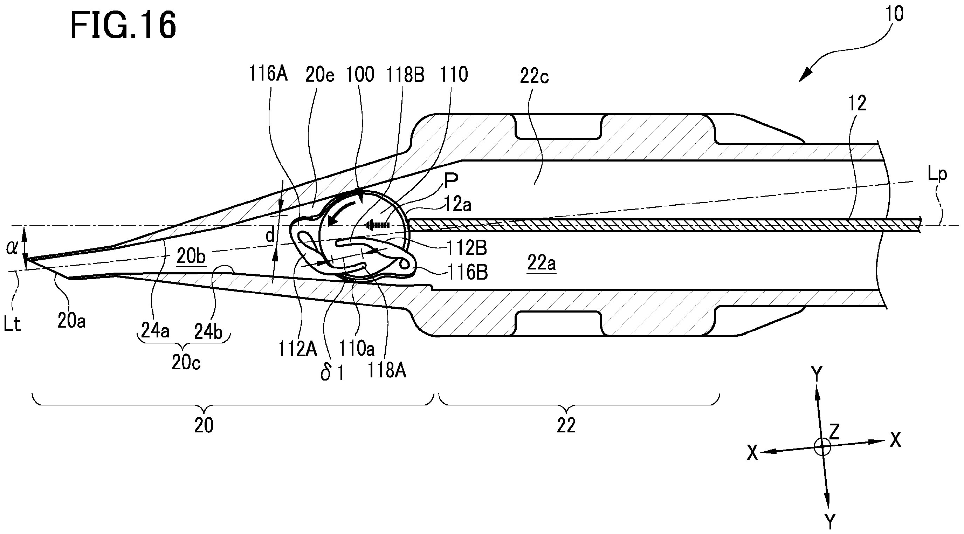

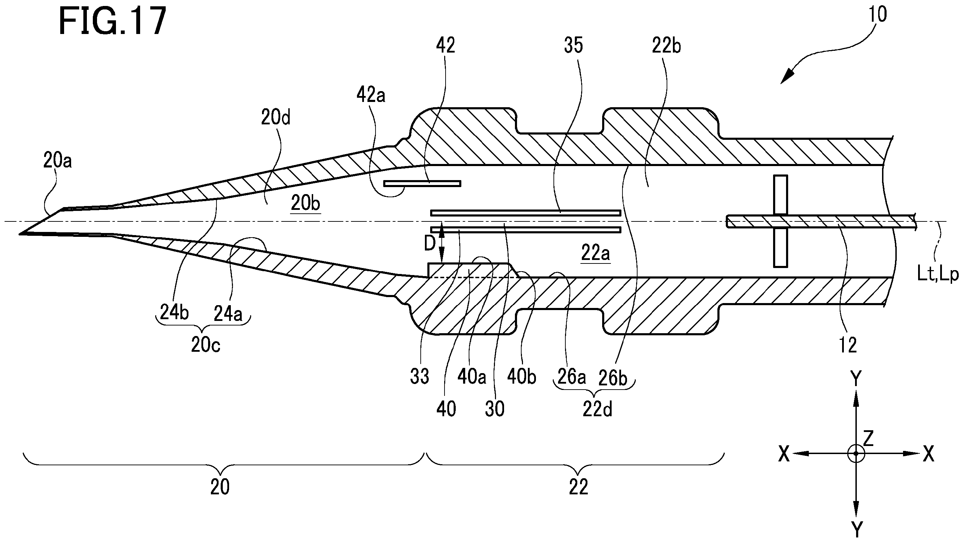

Embodiments of the present disclosure will now be explained below with reference to the accompanying drawings. A modified example of the second example shown in Fig. 16 and the second embodiment shown in Figs. 17 to 26 are examples which only contribute to understanding the present invention and do not form part of the claimed invention.

### (First Embodiment)

### <First Example>

A first example of a first embodiment is now explained.

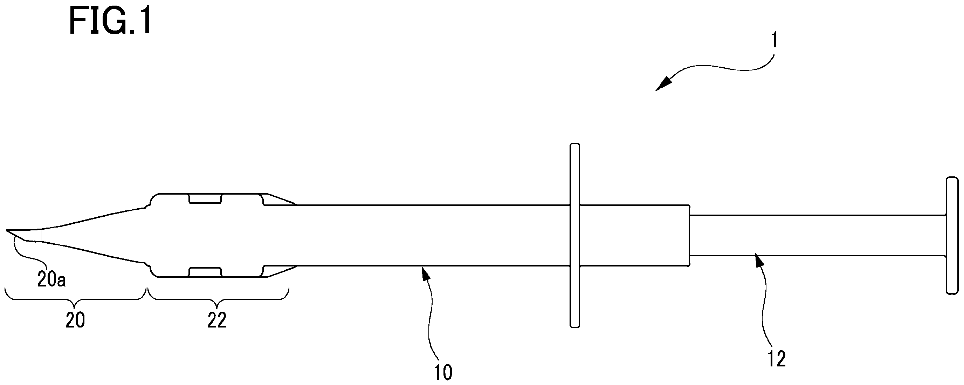

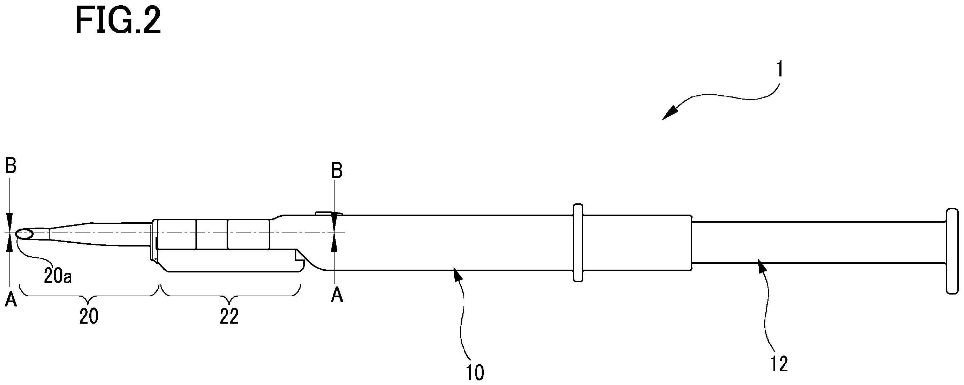

A configuration of an injector 1 embodying an intraocular lens (IOL) injection instrument is explained first. As shown in FIGs. 1 and 2, the injector 1 of the present embodiment includes a cylinder body (hereinafter, referred as a "body") 10, a plunger 12 (a push member). The body 10 is provided with an injection part 20, a setting part 22. The injector 1 with this configuration is, for example, formed by injection molding using resin material (for example, polypropylene) or the like. As one alternative, the injector 1 may be formed by cutting or machining resin. Further, as another alternative, the injector 1 may be a cartridge-type in which the injection part 20 is replaceable.

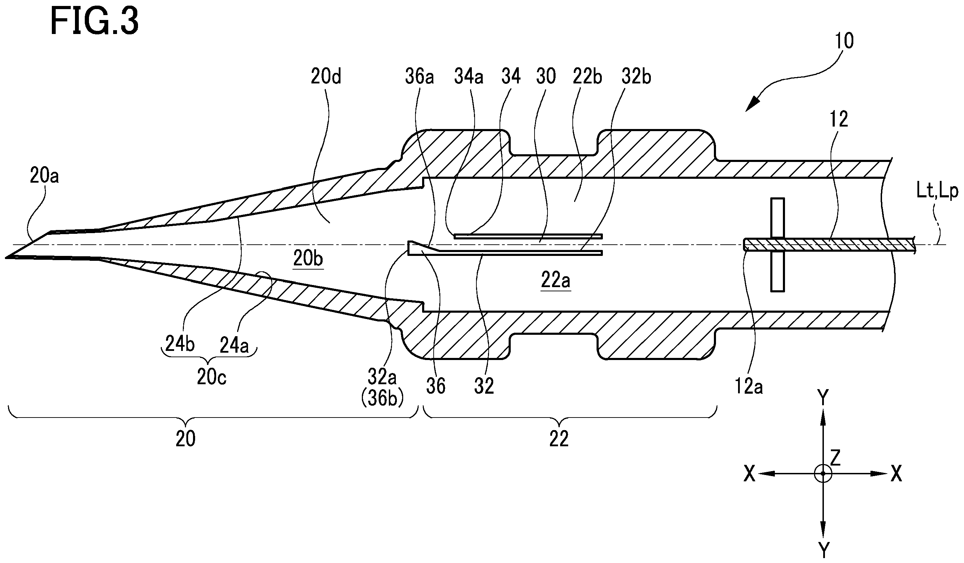

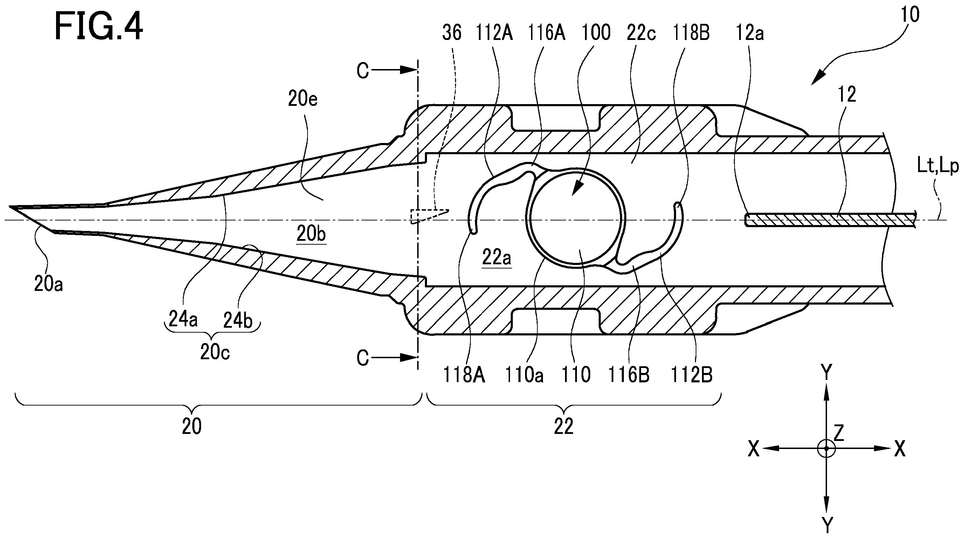

The injection part 20 is formed in a cylindrical hollow shape. As shown in FIGs. 3 and 4, the injection part 20 includes a passage 20b (a front-side passage). The passage 20b has a space made to be gradually narrow as coming close to a tip end 20a of the injection part 20 so that an intraocular lens (IOL) 100 is folded. In short, a passage area gradually decreases toward the tip end 20a. Herein, the passage area represents a sectional area of the passage 20b in a section orthogonal to a push-out direction (a leftward direction in FIGs. 3 and 4) of the IOL 100.

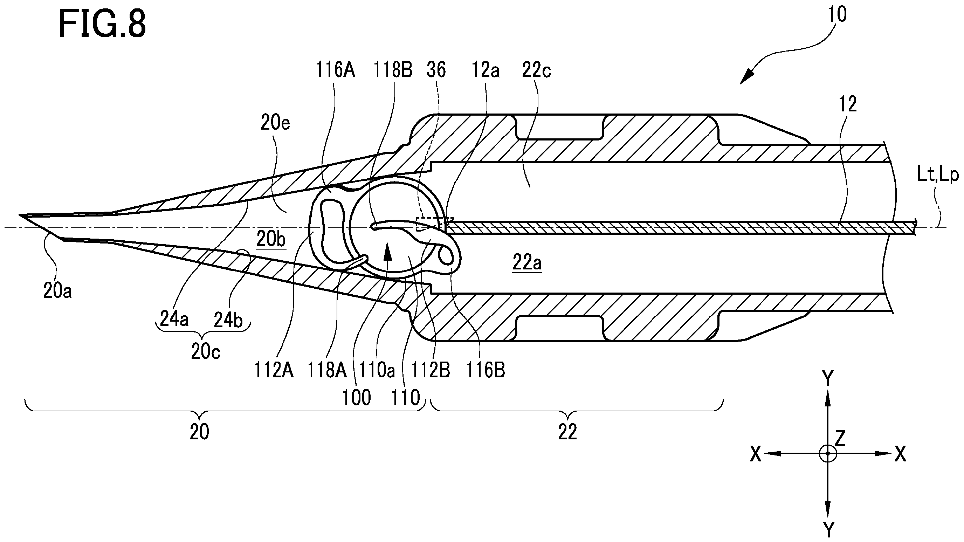

Further, the tip end 20a of the injection part 20 is formed with a cutout (bevel) for feeding the IOL 100 outside. While the IOL 100 is passing through the passage 20b, the IOL 100 is folded into a tiny piece along inner wall surfaces 20c (front-side inner wall surfaces) and then fed out from the cutout of the tip end 20a, so that the IOL 100 is injected into an eye. The inner wall surfaces 20c (a first inner wall surface 24a (a first front-side inner wall surface) and a second inner wall surface 24b (a second front-side inner wall surface)) are formed on both sides (both ends) in a direction orthogonal to a central axis Lt of the passage 20b, the direction being parallel to a radial direction of an optical part 110 when the IOL 100 is placed in the passage 20b (see FIG. 8).

Further, in the present embodiment, the first inner wall surface 24a and the second inner wall surface 24b are formed (inclined) symmetrically with respect to the central axis Lt of the passage 20b.

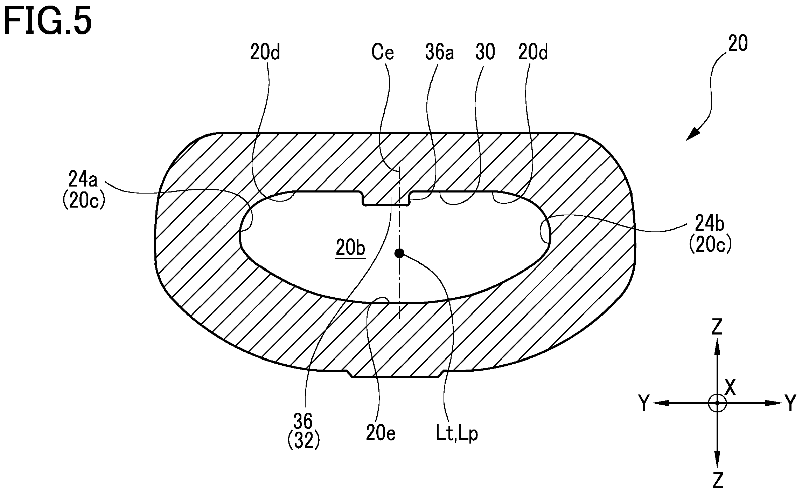

As shown in FIG. 5, a ceiling surface 20d (a first axial surface) and a bottom surface 20e (a second axial surface) are formed on both sides (both ends) in a direction orthogonal to the central axis Lt of the passage 20b, the direction being parallel to a central axis (an optical axis) L of the optical part 110 when the IOL 100 is placed in the passage 20b. Further, the bottom surface 20e has the shape of an outward curve (a downward curve in FIG. 5) in the passage 20b to be in a recess-like shape. In other words, the bottom surface 20e is defined as a surface facing a first protrusion 32 (a protruding portion 36) mentioned below in the passage 20b, and thus the bottom surface 20e is recessed in a protruding direction of the first protrusion 32.

Herein, as shown in FIGs. 3 to 5, axes X, Y, and Z which are orthogonally intersecting one another are assumed, and a direction indicated with the axis X is defined as a direction parallel to the push-out direction of the IOL 100 (a direction of the central axis Lt of the passage 20b and a passage 22a, a direction of a push-out axis Lp). Based on this assumption, the passage 20b is surrounded by the two inner wall surfaces 20c (the first inner wall surface 24a and the second inner wall surface 24b) formed on both sides in an axis Y direction, the ceiling surface 20d and the bottom surface 20e formed on both sides in an axis Z direction (see FIG. 5).

In the present embodiment, the central axis Lt of the passage 20b is coaxial with the push-out axis Lp.

The setting part 22 is located rearward (in a rightward direction in FIGs. 1 to 4) of the injection part 20 in the push-out direction of the plunger 12. In a passage 22a (a rear-side passage) formed in the setting part 22, the IOL 100 which is ready to be pushed out by the plunger 12 is placed (see FIG. 4).

The setting part 22 includes, as shown in FIGs. 3 and 4, the passage 22a, a ceiling surface 22b, a bottom surface 22c, a guide portion 30, the first protrusion 32 (a first guide protrusion), a second protrusion 34 (a second guide protrusion). The guide portion 30 is provided on the ceiling surface 22b to guide the push-out direction of the plunger 12.

The first protrusion 32 and the second protrusion 34 are formed along the push-out direction of the plunger 12. The first protrusion 32 constitutes one wall surface (a lower side of the paper in FIG. 3) of the guide portion 30 and protrudes from the ceiling surface 20d and the ceiling surface 22b toward the bottom surface 20e and the bottom surface 22c (toward a front side of the paper in FIG. 3). The second protrusion 34 constitutes the other wall surface (an upper side of the paper in FIG. 3) of the guide portion 30 and protrudes from the ceiling surface 22b toward the bottom surface 22c (toward the front side of the paper in FIG. 3).

In the present embodiment, the first protrusion 32 is provided with a protruding portion 36 (a lens control mechanism). The protruding portion 36 is provided in the first protrusion 32 close to the tip end 20a of the injection part 20. As shown in FIG. 3, the protruding portion 36 is formed to protrude more inwardly toward an inside of the guide portion 30 than a guide face 32b facing the guide portion 30 in the first protrusion 32. Further, the protruding portion 36 is formed in an inclined shape (a tapered shape) such that a guide face 36a facing the guide portion 30 gradually protrudes toward the inside of the guide portion 30 as coming close to the tip end 20a of the injection part 20. Namely, a protruding amount of the protruding portion 36 to the inside of the guide portion 30 is gradually increased as coming close to the tip end 20a of the injection part 20. Incidentally, the guide face 36a constitutes a face to guide the plunger 12 which will be mentioned later. As one alternative configuration of the above, the protruding portion 36 may be formed separately from the first protrusion 32.

Further, as shown in FIG. 5, the protruding portion 36 protrudes from the ceiling surface 20d toward the bottom surface 20e in the passage 20b of the injection part 20. The protruding portion 36 protrudes also in the passage 22a of the setting part 22 from the ceiling surface 22b toward the bottom surface 22c.

Further in the present embodiment, a part of the protruding portion 36 spreads over a central part Ce in a direction (an axis Y direction) orthogonal to the central axis Lt of the passage 20b and parallel to the radial direction of the optical part 110 when the IOL 100 is placed in the passage 20b. To be specific, a front end portion 36b (an end portion on the tip end 20a side of the injection part 20) of the protruding portion 36 is formed to extend across the central part Ce from a point closer to the first inner wall surface 24a than the central part Ce to a point closer to the second inner wall surface 24b than the central part Ce in the axis Y direction (see FIG. 3).

As one alternative, the protruding portion 36 may be formed not across the central part Ce but only formed on the first inner wall surface 24a side.

Further, in the present embodiment, as shown in FIG. 3, the front end portion 34a of the second protrusion 34 close to the tip end 20a of the injection part 20 is formed more rearward than a front end portion 32a of the first protrusion 32 close to the tip end 20a in the push-out direction of the IOL 100.

Herein, names such as "the ceiling surface 20d," "the bottom surface 20e," "the ceiling surface 22b," and "the bottom surface 22c" are given as a matter of convenience, and those names do not strictly designate an upper and lower direction of the injector 1. For instance, the bottom surface 20e is not always located on a lower side of the IOL 100. Specifically, the upper and lower sides of the injector 1 may be changed in each occasion of conveyance, filling a viscoelastic substance into the injector 1, injection of the IOL 100 into the eye.

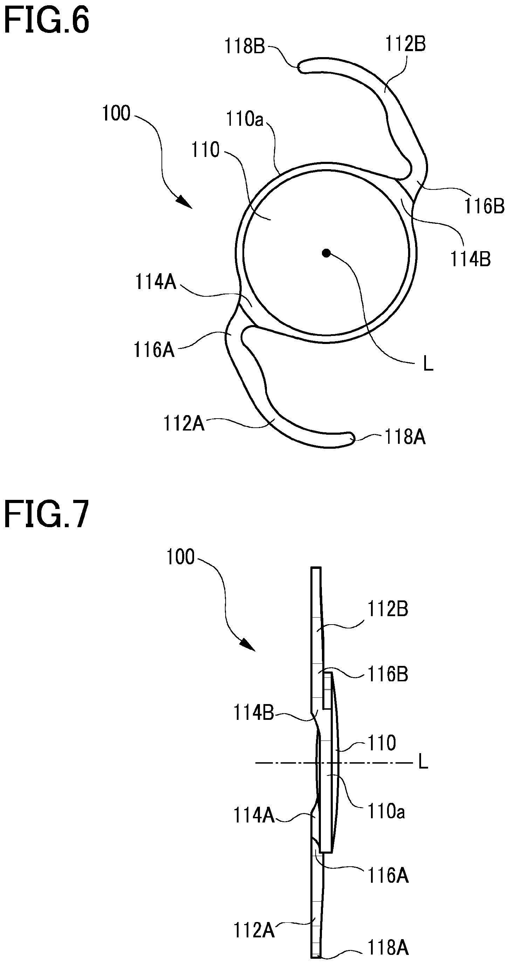

Next, the IOL 100 used in the present embodiment is explained. As shown in FIGs. 6 and 7, the IOL 100 used in the present embodiment is a one-piece IOL integrally formed of the optical part 110 and a pair of support parts including a front-side support part 112A (a first support part) and a rear-side support part 112B (a second support part), which are made of soft material.

The optical part 110 is formed in a disk-like shape. The front-side support part 112A and the rear-side support part 112B both extends outward from an outer circumferential part 110a of the optical part 110, and are formed in point-symmetrical positions with reference to a center of the optical part 110. The front-side support part 112A is provided with a root portion 116A which is connected to the outer circumferential part 110a of the optical part 110 via a connecting portion 114A, and a distal end portion 118A is open (namely, the distal end portion 118A is a free end). Further, the rear-side support part 112B is provided with a root portion 116B which is connected to the outer circumferential part 110a of the optical part 110 via a connecting portion 114B, and a distal end portion 118B is open.

Next, a method of injecting the IOL 100 into the eye by use of the injector 1 is explained to describe an operation of the injector 1.

An operator who operates the injector 1 firstly places the IOL 100 in the passage 22a of the setting part 22 as shown in the above mentioned FIG. 4 in such a manner that the front-side support part 112A is positioned ahead of (closer than) the optical part 110 toward the tip end 20a of the injection part 20. The operator then pushes the rear-side support part 112B of the IOL 100 by the plunger 12. Thus, the root portion 116B of the rear-side support part 112B is curved and the rear-side support part 112B is folded inside the outer circumferential part 110a of the optical part 110 and placed on top of the optical part 110.

Subsequently, the operator further pushes the outer circumferential part 110a of the optical part 110 of the IOL 100 by the plunger 12, and thus the IOL 100 is pushed toward the tip end 20a of the injection part 20. At this time, a leading end 12a (a contact portion with the IOL 100) of the plunger 12 is directed to move on the push-out axis Lp. Then, as shown in FIG. 8, in the passage 20b, the outer circumferential part 110a of the optical part 110 of the IOL 100 comes to contact with the inner wall surfaces 20c, and the optical part 110 is folded as valley-folded toward the bottom surface 20e (toward a rear side of the paper in FIG. 8).

At the same time with the above, the front-side support part 112A of the IOL 100 comes to contact with the inner wall surfaces 20c and the root portion 116A is gradually curved by the stress applied by the inner wall surfaces 20c and folded toward the optical part 110. Subsequently, as shown in FIG. 8, the distal end portion 118A of the front-side support part 112A is placed inside the outer circumferential part 110a of the optical part 110, so that the front-side support part 112A gets in the inside (inside the outer circumferential part 110a) of the optical part 110. In other words, the distal end portion 118A of the front-side support part 112A gets in a space defined by the optical part 110 and the ceiling surface 20d. Further at this time, the root portion 116A of the front-side support part 112A is placed in a position closer to the first inner wall surface 24a than the central axis Lt of the passage 20b, and on the other hand, the root portion 116B of the rear-side support part 112B is placed in a position closer to the second inner wall surface 24b than the central axis Lt of the passage 20b.

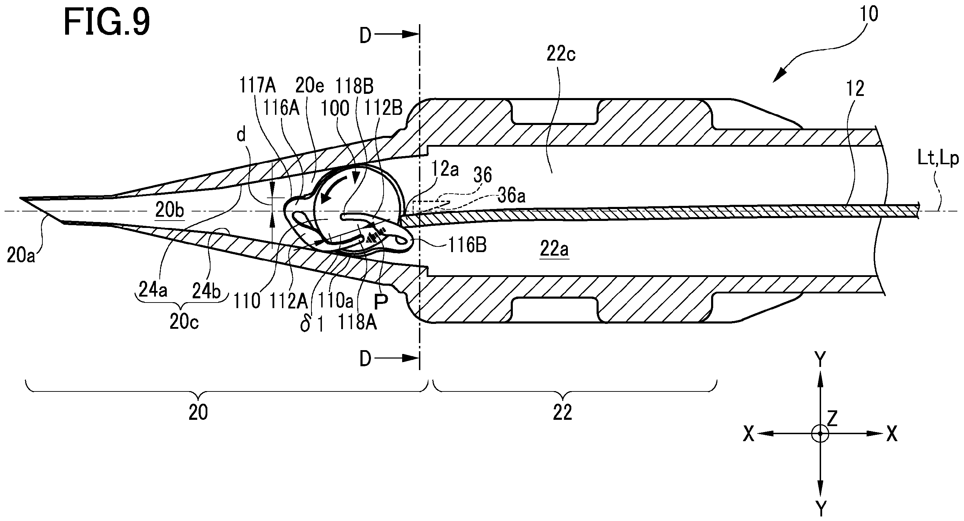

Afterwards, the IOL 100 is further pushed toward the tip end 20a of the injection part 20, and as shown in FIG. 9, the IOL 100 is rotated (counterclockwise, in a direction indicated with a bold arrow in FIG. 9) in such a manner that the root portion 116A of the front-side support part 112A moves away from the first inner wall surface 24a and comes close to the push-out axis Lp (the central axis Lt of the passage 20b) in a circumferential direction of the IOL 100. Thus, the front-side support part 112A gets in further inside of the optical part 110. A detailed explanation of a process that the front-side support part 112A gets in further inside of the optical part 110 shown in FIG. 9 from the state shown in FIG. 8 will be given later.

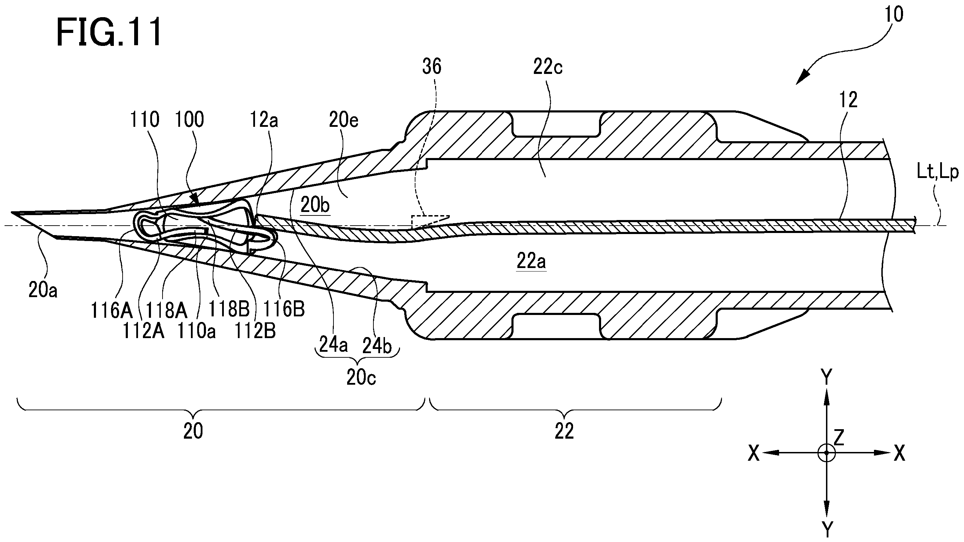

Subsequently, the IOL 100 is further pushed toward the tip end 20a of the injection part 20, and as shown in FIG. 11, the root portion 116A is further curved and the front-side support part 112A is folded and kept to be placed on top of the optical part 110 facing the ceiling surface 20d. Furthermore, the optical part 110 is folded into a tiny piece as valley-folded toward a rear side of the paper in FIG. 11. The rear-side support part 112B is also kept to be placed on top of the optical part 110 facing the ceiling surface 20d.

Subsequently, while the IOL 100 is further pushed toward the tip end 20a of the injection part 20, the front-side support part 112A and the rear-side support part 112B are kept to be placed on top of the optical part 110 facing the ceiling surface 20d and the optical part 110 is folded into a tiny piece as valley-folded toward the bottom surface 20e, and subsequently, the IOL 100 is pushed out of the injection part 20 through the tip end 20a of the injection part 20 and moved into the eye. In this manner, the folded IOL 100 is injected into the eye.

It is now explained in detail a process that the front-side support part 112A further gets in the inside of the optical part 110 as shown in FIG. 9 from the state shown in FIG. 8.

Before explanation of the present embodiment, a comparative example in which the first protrusion 32 is not provided with the protruding portion 36 is explained.

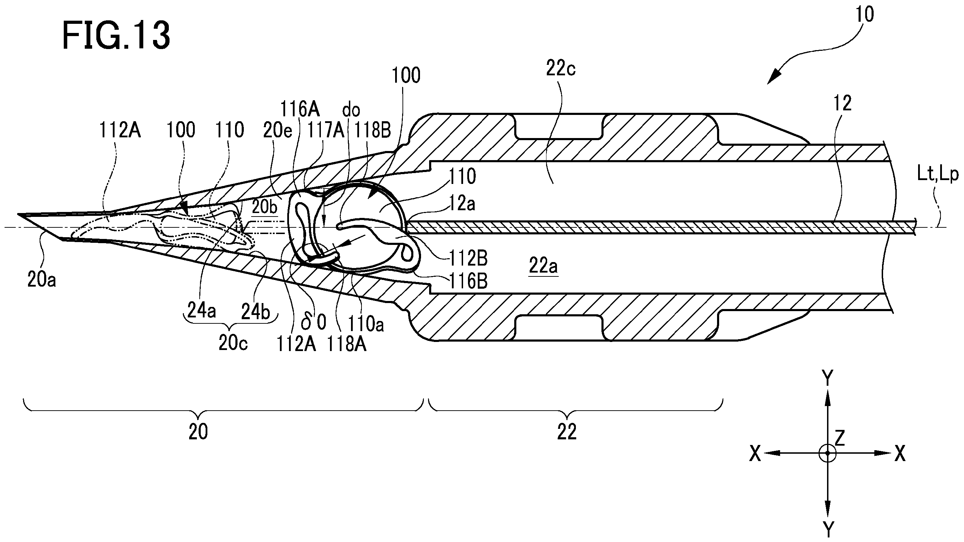

In the comparative example, when the IOL 100 is pushed toward the tip end 20a of the injection part 20 from the state shown in the above mentioned FIG. 8, as shown in FIG. 13, the leading end 12a of the plunger 12 is kept to move forward on the push-out axis Lp.

During that time, the IOL 100 is pushed toward the tip end 20a of the injection part 20 with a state in which an entering amount δ0 of the front-side support part 112A getting in the inside of the optical part 110 is made small. In this comparative example, a distance do is defined as a direct distance from an edge portion 117A of the root portion 116A of the front-side support part 112A facing the first inner wall surface 24a to the push-out axis Lp. The entering amount δ0 of the front-side support part 112A is small, and therefore, if the IOL 100 moving forward in the passage 20b is oscillated, for example, there is a possibility that the front-side support part 112A which has got in the inside of the optical part 110 comes out of the optical part 110.

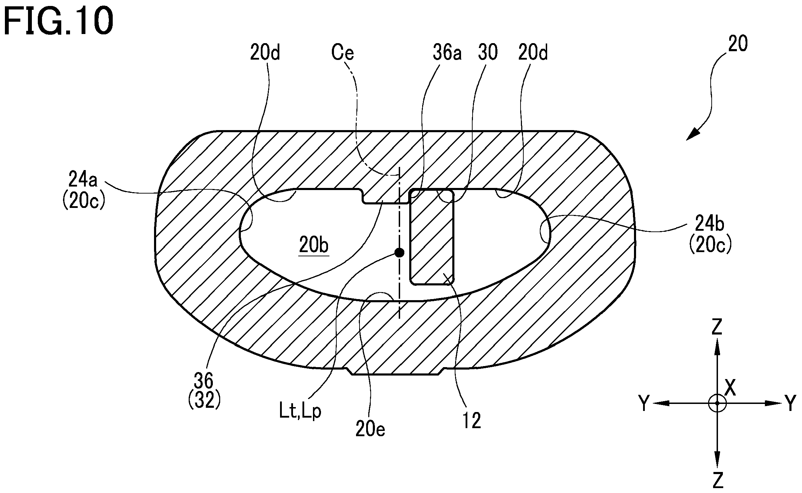

On the other hand, in the present embodiment, when the IOL 100 is pushed toward the tip end 20a of the injection part 20 from the state shown in the above mentioned FIG. 8, as shown in FIG. 9, the leading end 12a of the plunger 12 moves toward the second inner wall surface 24b. Namely, as shown in FIGs. 9 and 10, the plunger 12 is guided by the guide face 36a of the protruding portion 36, and thereby the leading end 12a of the plunger 12 comes off the push-out axis Lp and moves closer to the second inner wall surface 24b than the push-out axis Lp. In this manner, the protruding portion 36 directs the leading end 12a of the plunger 12 toward the second inner wall surface 24b.

When the leading end 12a of the plunger 12 moves toward the second inner wall surface 24b as mentioned above, a push-out force P applied to the IOL 100 from the plunger 12 acts on the second inner wall surface 24b side of the IOL 100. The second inner wall surface 24b has a uniform frictional force over the whole surface, but the push-out force P applied to the optical part 110 increases a pushing force to push the optical part 110 against the second inner wall surface 24b, thereby increasing a frictional force generated between the outer circumferential part 110a of the optical part 110 and the second inner wall surface 24b (a reaction force that the outer circumferential part 110a of the optical part 110 receives from the second inner wall surface 24b). Therefore, a contact portion of the outer circumferential part 110a of the optical part 110 contacting the second inner wall surface 24b becomes hard to move toward the tip end 20a of the injection part 20. On the other hand, a frictional force generated between the outer circumferential part 110a of the optical part 110 and the first inner wall surface 24a (a reaction force that the outer circumferential part 110a of the optical part 110 receives from the first inner wall surface 24a) is decreased, and thus a contact portion of the outer circumferential part 110a of the optical part 110 contacting the first inner wall surface 24a becomes easy to move toward the tip end 20a of the injection part 20. As a result, the IOL 100 is rotated in the circumferential direction of the optical part 110 such that the root portion 116A of the front-side support part 112A moves away from the first inner wall surface 24a and comes close to the push-out axis Lp.

At that time, a direct distance from the edge portion 117A on the first inner wall surface 24a side of the root portion 116A in the front-side support part 112A to the push-out axis Lp is defined as a distance d, and this distance d is smaller than the above mentioned distance do. Further, an entering amount δ1 of the front-side support part 112A getting in the inside of the optical part 110 is large (larger than the above mentioned amount δ0).

As mentioned above, the entering amount δ1 of the front-side support part 112A is large, and therefore, even if the IOL 100 is oscillated during movement in the passage 20b, for example, the front-side support part 112A having got in the inside of the optical part 110 is prevented from coming out of the optical part 110.

Subsequently, the IOL 100 is further pushed toward the tip end 20a of the injection part 20, as shown in FIG. 11, the plunger 12 is curved and the leading end 12a of the plunger 12 returns onto the push-out axis Lp. After that, the IOL 100 of a favorable folded shape is injected outside the injection part 20. At this time, the IOL 100 may only have to be injected outside the injection part 20, and the leading end 12a of the plunger 12 does not have to come out of the tip end 20a of the injection part 20, for example. Further, for example, in a case that the leading end 12a of the plunger 12 comes out of the tip end 20a, a part of the plunger 12 that has come out may not be in a position along the push-out axis Lp.

As mentioned above, in the present embodiment, the protruding portion 36 directs the leading end 12a of the plunger 12 to the second inner wall surface 24b so that an orientation of the IOL 100 in the circumferential direction of the optical part 110 is controlled.

In the above explanation, as shown in FIG. 9, after the distal end portion 118A of the front-side support part 112A is placed inside the outer circumferential part 110a of the optical part 110 (see FIG. 8), the orientation of the IOL 100 in the circumferential direction of the optical part 110 is controlled. However, the timing is not limited to the above, and a position of the protruding portion 36 may be varied or other measures may be taken to control the orientation of the IOL 100 in the circumferential direction of the optical part 110 simultaneously with the time when the distal end portion 118A of the front-side support part 112A is placed inside the outer circumferential part 110a of the optical part 110.

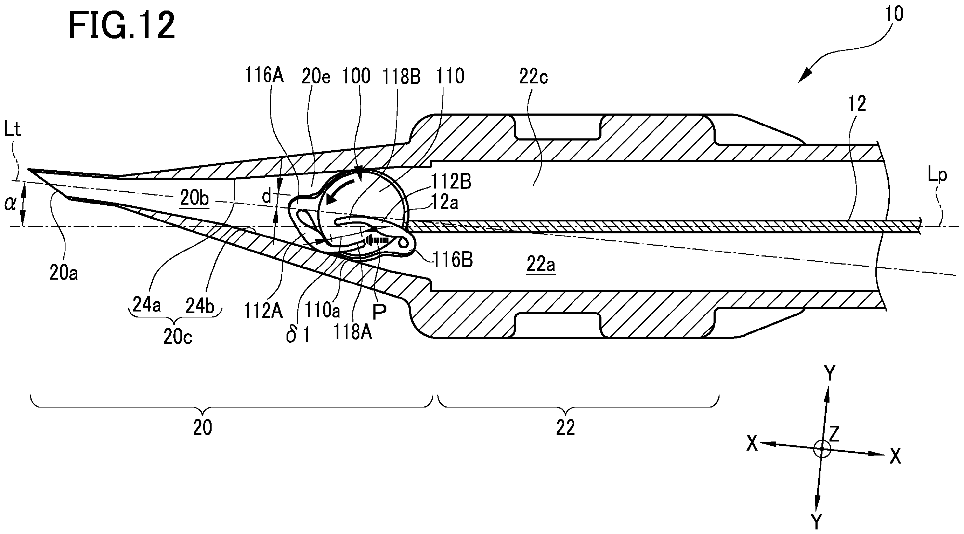

As one modified example, as shown in FIG. 12, the passage 20b may be configured as an inclined passage (a lens control mechanism) in which the central axis Lt of the passage 20b intersects the push-out axis Lp. To be more specific, the central axis Lt of the passage 20b may intersect and be slanted with respect to the push-out axis Lp to the radial direction (the axis Y direction) of the optical part 110 when the IOL 100 is placed in the passage 20b. As one example, an inclination angle α of the central axis Lt of the passage 20b with respect to the push-out axis Lp is 5°. Further, in FIG. 12, a direction of the central axis Lt of the passage 20b is defined as the axis X direction.

Thus, each inclination of the first inner wall surface 24a and the second inner wall surface 24b with respect to the push-out axis Lp are differentiated from each other. Specifically, the inclination of the second inner wall surface 24b with respect to the push-out axis Lp is larger than that of the first inner wall surface 24a with respect to the push-out axis Lp. Further, the second inner wall surface 24b intersects the push-out axis Lp.

In the modified example, when the leading end 12a of the plunger 12 is pushed to move along the push-out axis Lp, the leading end 12a of the plunger 12 is directed toward the second inner wall surface 24b. By this movement of the leading end 12a of the plunger 12 toward the second inner wall surface 24b, the IOL 100 is rotated as similarly to the above mentioned example shown in FIG. 9 (see FIG. 12).

In the modified example, too, the first inner wall surface 24a and the second inner wall surface 24b are formed (inclined) symmetrically with respect to the central axis Lt of the passage 20b. In the example shown in FIG. 12, the whole passage 20b is configured as an inclined passage, but as another modified example, only a part of the passage 20b may be configured as the inclined passage. For example, a part of the passage 20b closer to the tip end 20a of the injection part 20 than a predetermined point may be configured as the inclined passage. Namely, the central axis Lt of a part located closer to the tip end 20a of the injection part 20 than the predetermined point in the passage 20b intersects the push-out axis Lp, and a remaining part located opposite to the tip end 20a side from the predetermined point of the passage 20b may have the central axis Lt formed in parallel with the push-out axis Lp.

As another modified example, the IOL injection instrument 1 may be configured such that a first frictional force generated between the first inner wall surface 24a and the outer circumferential part 110a of the optical part 110 of the IOL 100 and a second frictional force generated between the second inner wall surface 24b and the outer circumferential part 110a of the optical part 110 of the IOL 100 are differentiated. In the present embodiment, a surface roughness of the second inner wall surface 24b is made rougher than a surface roughness of the first inner wall surface 24a so that the second frictional force is made larger than the first frictional force. Thus, the orientation of the IOL 100 in the circumferential direction of the optical part 110 is enabled to be controlled.

To be more specific, a contact portion of the outer circumferential part 110a in the optical part 110 contacting the second inner wall surface 24b is made hard to move toward the tip end 20a of the injection part 20. On the other hand, a contact portion of the outer circumferential part 110a in the optical part 110 contacting the first inner wall surface 24a is made easy to move toward the tip end 20a of the injection part 20. Therefore, the IOL 100 is rotated as similarly to the above mentioned FIG. 9. As a result, the entering amount δ1 of the front-side support part 112A can be increased.

As mentioned above, according to the present disclosure, when or after the IOL 100 is pushed toward the tip end 20a of the injection part 20 and the front-side support part 112A is folded and the distal end portion 118A is placed inside the outer circumferential part 110a of the optical part 110, the orientation of the IOL 100 in the circumferential direction of the optical part 110 is made to be controlled. Herein, "when or after the distal end portion 118A is placed inside the outer circumferential part 110a of the optical part 100" means the simultaneous timing with the time when the distal end portion 118A is placed inside the outer circumferential part 110a of the optical part 110 or the timing subsequent to the time when the distal end portion 118A is placed inside the outer circumferential part 110a of the optical part 110.

As a consequence, the entering amount δ1 of the front-side support part 112A getting in the inside of the optical part 110 can be controlled. Therefore, by controlling the entering amount δ1 of the front-side support part 112A to be increased, it is enabled to prevent the front-side support part 112A, which has once got in the inside of the optical part 110, from coming out of the optical part 110. Accordingly, when the IOL 100 is about to be injected, it is enabled to maintain a state in which the front-side support part 112A is folded and placed on top of the optical part 110. Thus, the IOL 100 can be appropriately deformed and injected.

Further, the protruding portion 36 is configured to direct the leading end 12a of the plunger 12 toward the second inner wall surface 24b so that the orientation of the IOL 100 in the circumferential direction of the optical part 110 is controlled. Thus, the IOL 100 is rotated in the circumferential direction of the optical part 110 such that the root portion 116A of the front-side support part 112A moves away from the first inner wall surface 24a and comes close to the push-out axis Lp. Accordingly, the entering amount δ1 of the front-side support part 112A can be effectively increased.

Further, the protruding portion 36 protrudes from the ceiling surface 20d toward the bottom surface 20e in the passage 20b. Thus, the leading end 12a of the plunger 12 is made to be in contact with the protruding portion 36, so that a moving direction of the leading end 12a of the plunger 12 can be controlled.

Further, the protruding portion 36 is located close to the tip end 20a of the injection part 20 in the axis X direction in the first protrusion 32 and protrudes more inwardly toward the guide portion 30 than the guide face 32b of the first protrusion 32 in the axis Y direction. In the present embodiment, the front end portion 36b of the protruding portion 36 is formed to extend in the axis Y direction from a position closer to the first inner wall surface 24a than the central part Ce in the passage 20b to a position closer to the second inner wall surface 24b than the central part Ce in the passage 20b across the central part Ce of the passage 20b. Thus, the leading end 12a of the plunger 12 is further easily moved toward the second inner wall surface 24b.

Further, the central axis Lt of the passage 20b may be inclined toward the first inner wall surface 24a with respect to the push-out axis Lp to the radial direction (in the axis Y direction) of the optical part 110 when the IOL 100 is placed in the passage 20b. Thus, when the leading end 12a of the plunger 12 is moved on the push-out axis Lp, the plunger 12 is made to move toward the second inner wall surface 24b. Therefore, the IOL 100 is rotated in the circumferential direction of the optical part 110 such that the root portion 116A of the front-side support part 112A moves away from the first inner wall surface 24a and comes close to the push-out axis Lp. Accordingly, the entering amount δ1 of the front-side support part 112A can be effectively increased.

Further, the first frictional force generated between the first inner wall surface 24a and the outer circumferential part 110a of the optical part 110 and the second frictional force generated between the second inner wall surface 24b and the outer circumferential part 110a of the optical part 110 may be differentiated. By enlarging the second frictional force larger than the first frictional force, the IOL 100 is rotated in the circumferential direction of the optical part 110 such that the root portion 116A of the front-side support part 112A moves away from the first inner wall surface 24a and comes close to the push-out axis Lp. As a result, the entering amount δ1 of the front-side support part 112A can be increased.

### <Second Example>

A second example of the first embodiment is now explained. In the present example, similar components are indicated with the same referential signs with the first example, and explanation thereof is omitted. Accordingly, the following explanation is made with a focus on the differences from the first example.

Depending on a shape and a hardness of the IOL 100 and a frictional coefficient of the first inner wall surface 24a, the leading end 12a of the plunger 12 may be moved toward the first inner wall surface 24a so as to rotate the IOL 100 in the circumferential direction of the optical part 110 such that the root portion 116A of the front-side support part 112A moves away from the first inner wall surface 24a and comes close to the push-out axis Lp.

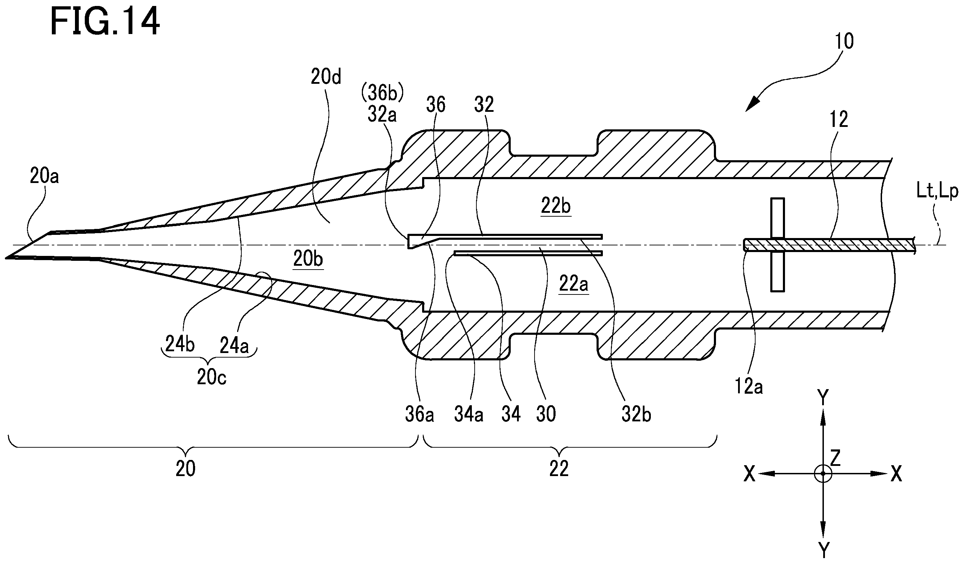

Therefore, in the present example, as shown in FIG. 14, a positional relation of the first protrusion 32 and the second protrusion 34 is inverted from the positional relation of the above mentioned first example (see FIG. 3). To be more specific, the second protrusion 34 constitutes one wall surface (a lower side in FIG. 14, a side of the first inner wall surface 24a) of the guide portion 30, and the first protrusion 32 constitutes the other wall surface (an upper side in FIG. 14, a side of the second inner wall surface 24b) of the guide portion 30.

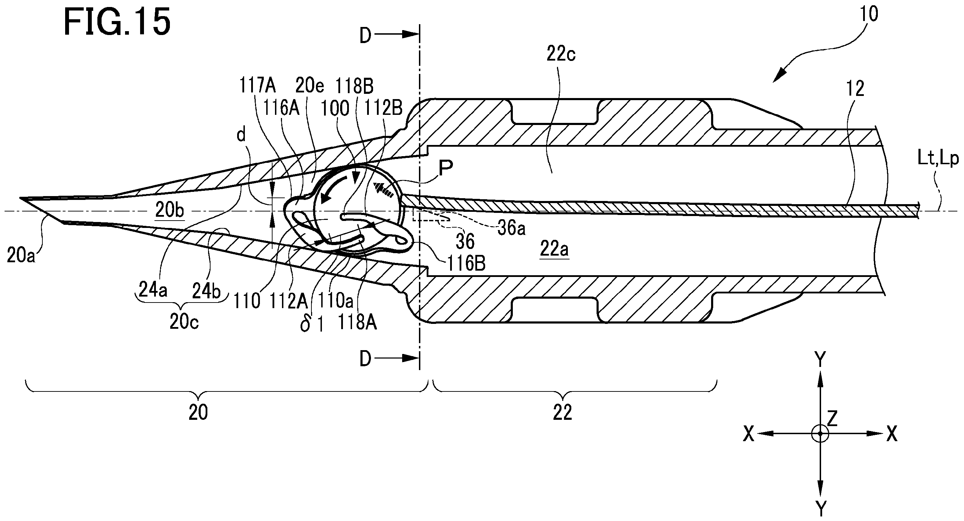

In the present example with this configuration, when the IOL 100 is pushed toward the tip end 20a of the injection part 20, as shown in FIG. 15, the leading end 12a of the plunger 12 is directed toward the first inner wall surface 24a. Namely, the plunger 12 is guided by the guide face 36a of the protruding portion 36 as shown in FIG. 15, and thus the leading end 12a of the plunger 12 comes off the push-out axis Lp and comes closer to the first inner wall surface 24a than the push-out axis Lp. Therefore, the protruding portion 36 directs the leading end 12a of the plunger 12 toward the first inner wall surface 24a.

When the leading end 12a of the plunger 12 is thus directed toward the first inner wall surface 24a, the push-out force P applied to the IOL 100 by the plunger 12 acts on the IOL 100 on the side of the first inner wall surface 24a. Herein, the first inner wall surface 24a has a uniform frictional force over the whole surface and the frictional force is small enough by an application of coating or the like. Accordingly, the IOL 100 is rotated in the circumferential direction of the optical part 110 by the push-out force P such that the root portion 116A of the front-side support part 112A moves away from the first inner wall surface 24a and comes close to the push-out axis Lp.

Thus, in the present example, the protruding portion 36 directs the leading end 12a of the plunger 12 to move toward the first inner wall surface 24a, and thereby the orientation of the IOL 100 in the circumferential direction of the optical part 110 is controlled.

As a result, the IOL 100 is rotated in the circumferential direction of the optical part 110 such that the root portion 116A of the front-side support part 112A moves away from the first inner wall surface 24a and comes close to the push-out axis Lp. Therefore, the entering amount δ1 of the front-side support part 112A can be effectively increased.

Further, as a modified example of the second example, as shown in FIG. 16, the passage 20b may be configured as an inclined passage (a lens control mechanism) of which the central axis Lt of the passage 20b intersects the push-out axis Lp. To be specific, an inclination of the first inner wall surface 24a with respect to the push-out axis Lp is made larger than that of the second inner wall surface 24b with respect to the push-out axis Lp. Thus, the first inner wall surface 24a intersects the push-out axis Lp.

In the modified example, when the leading end 12a of the plunger 12 is moved forward on the push-out axis Lp, the leading end 12a of the plunger 12 is directed toward the first inner wall surface 24a. When the leading end 12a of the plunger 12 is directed toward the first inner wall surface 24a in this manner, the IOL 100 is rotated as similarly to the above mentioned FIG. 15 (see FIG. 16).

In this manner, the central axis Lt of the passage 20b may be inclined with respect to the push-out axis Lp toward the second inner wall surface 24b to the radial direction (in the axis Y direction) of the optical part 110 when the IOL 100 is placed in the passage 20b. Thus, by movement of the leading end 12a of the plunger 12 on the push-out axis Lp, the plunger 12 can be moved toward the first inner wall surface 24a. As a result, the IOL 100 is rotated in the circumferential direction of the optical part 110 such that the root portion 116A of the front-side support part 112A moves away from the first inner wall surface 24a and comes close to the push-out axis Lp. Accordingly, the entering amount δ1 of the front-side support part 112A can be effectively increased.

The technique illustrated in the above embodiment may be adopted for an IOL injection instrument of a preset type in which an IOL 100 is loaded inside in advance and for an IOL injection instrument which is to be supplied or shipped without preloading the IOL 100. Further, the IOL 100 illustrated in the above embodiment is a one-piece IOL in which the optical part 110, the front-side support part 112A, and the rear-side support part 112B are integrally formed. Alternatively, the technique illustrated in the above embodiment may be applied to an IOL injection instrument for injecting an intraocular lens other than a one-piece type (for example, a 3-piece IOL). Further, the root portions 116A and 116B of the IOL 100 may be directly connected to the outer circumferential part of the optical part 110 without providing the connecting portions 114A and 114B.

### (Second Embodiment)

A second embodiment of the present disclosure is now explained. In the explanation, similar components are indicated with the same referential signs with the first embodiment, and explanation thereof is omitted. Accordingly, the following explanation is made with a focus on the differences from the first embodiment.

In the present embodiment, the setting part 22 is, as shown in FIGs. 17 to 19, provided with the passage 22a, the ceiling surface 22b, the bottom surface 22c, the inner wall surface 22d (a rear-side inner wall surface), the guide portion 30, a first guide protrusion 33, a second guide protrusion 35, a first protrusion 40, a second protrusion 42.

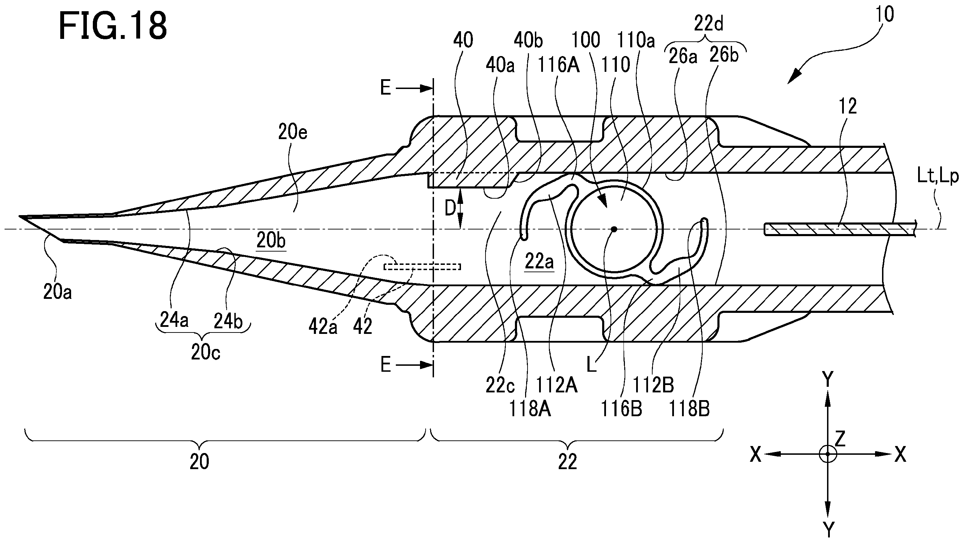

As shown in FIG. 18, the inner wall surfaces 22d (the first inner wall surface 26a (a first rear-side inner wall surface) and the second inner wall surface 26b (a second rear-side inner wall surface)) are formed on both sides in a direction orthogonal to the central axis Lt of the passage 22a, the direction being parallel to the radial direction of the optical part 110 when the IOL 100 is placed in the passage 22a. Specifically, the first inner wall surface 26a is provided on a side where the root portion 116A of the front-side support part 112A is placed in the passage 22a with respect to the central axis Lt of the passage 22a when the IOL 100 is placed therein. On the other hand, the second inner wall surface 26b is provided on a side where the root portion 116B of the rear-side support part 112B is placed in the passage 22a with respect to the central axis Lt of the passage 22a when the IOL 100 is placed therein.

The guide portion 30 is formed on the ceiling surface 22b to guide the push-out direction of the plunger 12. The first guide protrusion 33 and the second guide protrusion 35 are formed along the push-out direction of the plunger 12. The first guide protrusion 33 constitutes one wall surface (a lower side in FIG. 17) of the guide portion 30 and protrudes from the ceiling surface 22b toward the bottom surface 22c (toward a front side of the paper in FIG. 17). The second guide protrusion 35 constitutes the other wall surface (an upper side in FIG. 17) of the guide portion 30 and protrudes from the ceiling surface 22b toward the bottom surface 22c (toward the front side of the paper in FIG. 17).

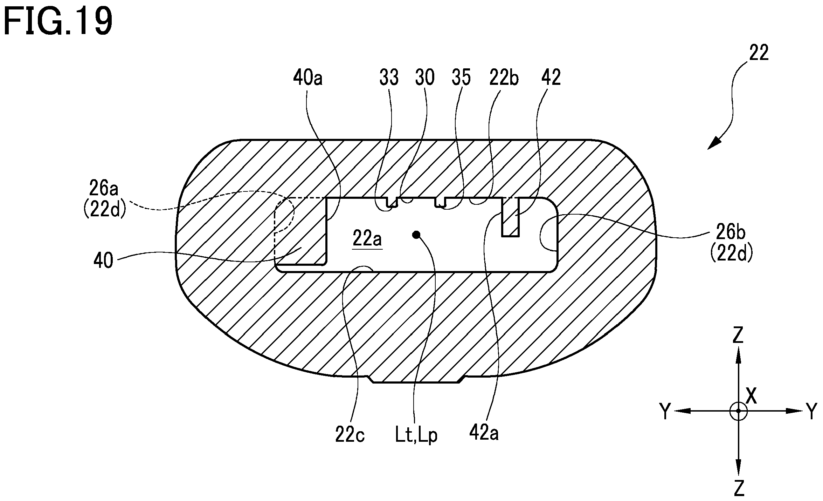

In the present embodiment, the first protrusion 40 is, as shown in FIG. 17, formed to protrude from the first inner wall surface 26a toward the central axis Lt of the passage 22a. Further, as shown in FIG. 19, the first protrusion 40 is formed to protrude from the ceiling surface 22b toward the bottom surface 22c. In an example shown in FIG. 17, the first protrusion 40 is formed to be in an elongated shape extending in parallel with the central axis Lt of the passage 22a in an end portion of the passage 22a close to the injection part 20.

Further, the first protrusion 40 has an inside surface 40a (a first contact portion, an optical contact portion) and a slope portion 40b. The inside surface 40a is formed on an end face of the first protrusion 40 which protrudes from the first inner wall surface 26a toward the central axis Lt of the passage 22a. In short, the inside surface 40a of the first protrusion 40 is formed in a position closer to the central axis Lt of the passage 22a than the first inner wall surface 26a, namely, formed between the first inner wall surface 26a and the central axis Lt of the passage 22a. In the example shown in FIG. 17, the inside surface 40a of the first protrusion 40 is provided between the first inner wall surface 26a and the first guide protrusion 33.

In the present embodiment, a distance D between the inside surface 40a of the first protrusion 40 and the central axis Lt of the passage 22a is made to be shorter than a radius of the optical part 110. Further, the inside surface 40a of the first protrusion 40 is arranged in a position to be in contact with the outer circumferential part 110a of the optical part 110 when the IOL 100 is pushed out which will be explained later.

The slope portion 40b is provided on a rear end portion of the first protrusion 40 in the push-out direction of the plunger 12 such that a protruding amount of the slope portion 40b protruding from the first inner wall surface 26a to the central axis Lt of the passage 22a is gradually increased as coming close to a front side of the push-out direction of the plunger 12. Namely, the slope portion 40b is formed in an inclined direction with respect to the push-out direction of the plunger 12.

Further in the present embodiment, the second protrusion 42 is, as shown in FIG. 17, provided on the side closer to the central axis Lt of the passage 22a and the passage 20b than the second inner wall surface 26b and the second inner wall surface 24b. As shown in FIG. 19, the second protrusion 42 is formed to protrude from the ceiling surface 22b toward the bottom surface 22c. The second protrusion 42 is, as shown in FIG. 17, formed in an elongated shape extending in parallel with the central axis Lt of the passage 20b and the passage 22a in a portion including a boundary portion of the passage 22a and the passage 20b.

Further, the second protrusion 42 includes an inside surface 42a (a second contact portion, a support contact portion). The inside surface 42a is a surface formed on a side of the central axis Lt of the passage 22a in the second protrusion 42. The inside surface 42a of the second protrusion 42 is thus provided in a position closer to the central axis Lt of the passage 22a than the second inner wall surface 26b, namely, provided between the second inner wall surface 26b and the central axis Lt of the passage 22a. In the example shown in FIG. 17, a part of the inside surface 42a of the second protrusion 42 is positioned between the second inner wall surface 26b and the second guide protrusion 35.

In the present embodiment, the inside surface 42a of the second protrusion 42 is positioned to be in contact with the root portion 116B of the rear-side support part 112B on the second inner wall surface 26b side when or after the outer circumferential part 110a of the optical part 110 moves away from the inside surface 40a of the first protrusion 40 and when the front-side support part 112A and the optical part 110 are placed in the passage 20b during the push-out operation of the IOL 100 which will be mentioned later.

As indicated in FIGs. 17 to 19, the axes X, Y, and Z which are orthogonally intersecting one another are assumed, and a direction indicated with the axis X is defined as a direction parallel to the push-out direction of the IOL 100 (the central axis Lt direction of the passage 20b and the passage 22a, the push-out axis Lp direction). Based on this assumption, the passage 20b is surrounded by the two inner wall surfaces 20c (the first inner wall surface 24a and the second inner wall surface 24b) formed on both sides in the axis Y direction, the ceiling surface 20d and the bottom surface 20e formed on both sides in the axis Z direction. Further, the passage 22a is formed to be surrounded by the two inner wall surfaces 22d (the first inner wall surface 26a and the second inner wall surface 26b) formed on both sides in the axis Y direction, the ceiling surface 22b and the bottom surface 22c formed on both sides in the axis Z direction (see FIG. 19).

Next, a method of injecting the IOL 100 into the eye by use of the injector 1 is explained to describe an operation of the injector 1.

An operator who operates the injector 1 firstly places the IOL 100 in the passage 22a of the setting part 22 as shown in FIG. 18 such that the front-side support part 112A is positioned on the side closer to the tip end 20a of the injection part 20 than the optical part 110. At this time, in the example shown in FIG. 18, the root portion 116A of the front-side support part 112A is in contact with the first inner wall surface 26a of the setting part 22 and there is no clearance formed between the root portion 116A of the front-side support part 112A and the first inner wall surface 26a of the setting part 22.

The operator then pushes the rear-side support part 112B by the plunger 12. Thus, the root portion 116B of the rear-side support part 112B is curved and the rear-side support part 112B is folded inside the outer circumferential part 110a of the optical part 110 and placed on top of the optical part 110. In this manner, the IOL 100 is placed in the passage 22a with the rear-side support part 112B being folded inside the optical part 110.

Subsequently, the operator further pushes the outer circumferential part 110a of the optical part 110 by the plunger 12. Thus, the root portion 116A of the front-side support part 112A is brought into contact with the inside surface 40a of the first protrusion 40 while the IOL 100 is pushed toward the tip end 20a of the injection part 20.

After the root portion 116A of the front-side support part 112A moves away from the inside surface 40a of the first protrusion 40, the outer circumferential part 110a of the optical part 110 is guided by the slope portion 40b of the first protrusion 40, and the IOL 100 is pushed toward the tip end 20a of the injection part 20.

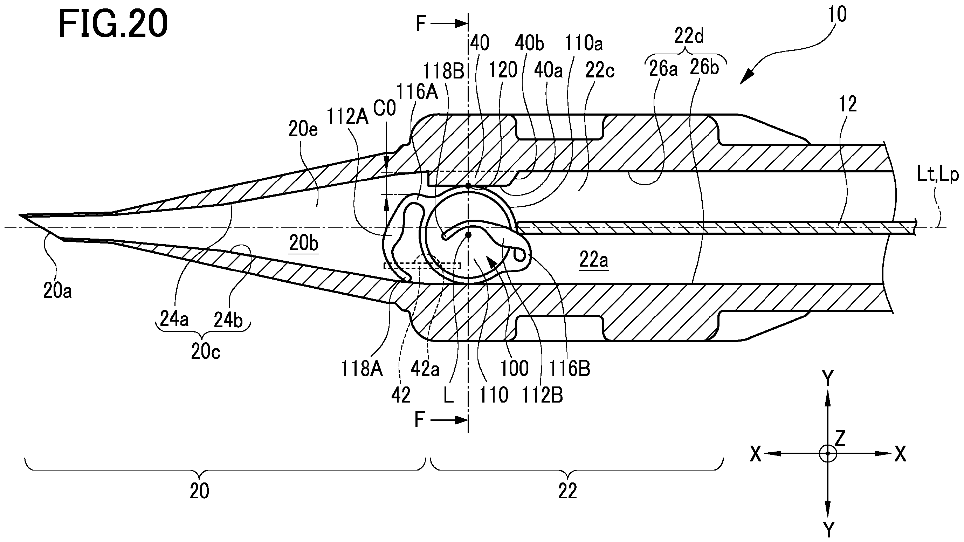

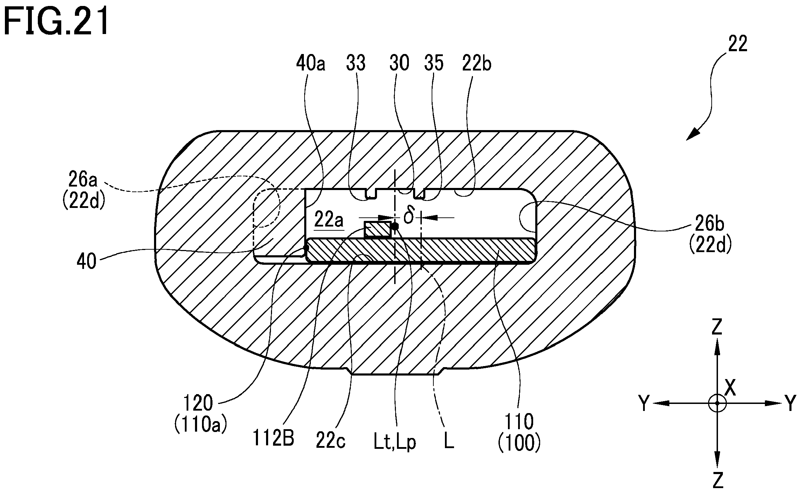

Subsequently, as shown in FIGs. 20 and 21, an edge portion 120 of the outer circumferential part 110a of the optical part 110 on the first inner wall surface 26a side comes to contact with the inside surface 40a of the first protrusion 40. At this time, the optical part 110 is pushed by the first protrusion 40 and directed toward the second inner wall surface 26b. Therefore, the optical axis L of the optical part 110 comes off the central axis Lt of the passage 22a and moves to a position apart from the central axis Lt by a distance δ (see FIG. 21) on the second inner wall surface 26b side. At the same time, the root portion 116A of the front-side support part 112A moves away from the first inner wall surface 26a. As a result, a clearance C0 is created between the root portion 116A of the front-side support part 112A and the first inner wall surface 26a. Accordingly, the root portion 116A of the front-side support part 112A is not interfered by the first inner wall surface 26a.

As it will be explained in detail later, formation of the clearance C0 thus makes it easy to subsequently form a clearance C1 between the root portion 116A of the front-side support part 112A and the first inner wall surface 24a when the IOL 100 is pushed into the passage 20b by the plunger 12.

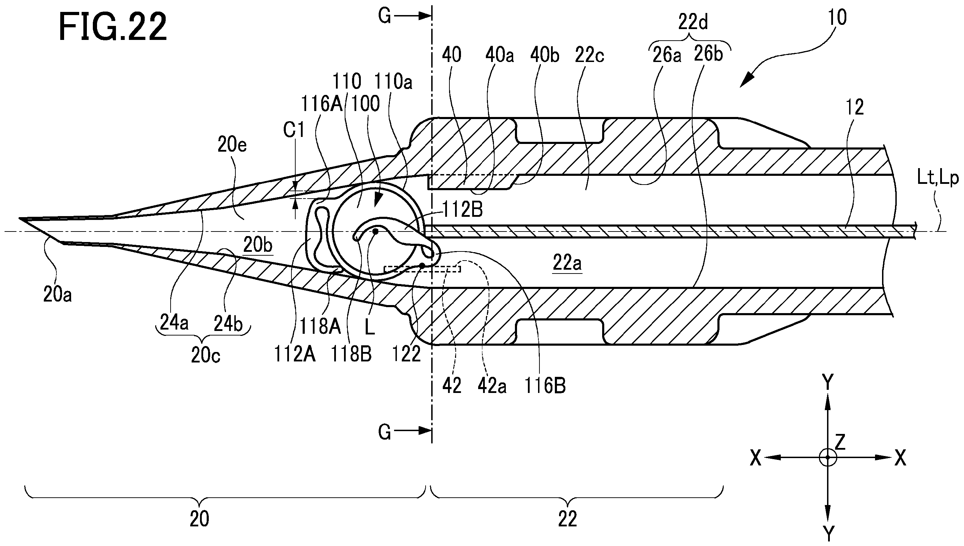

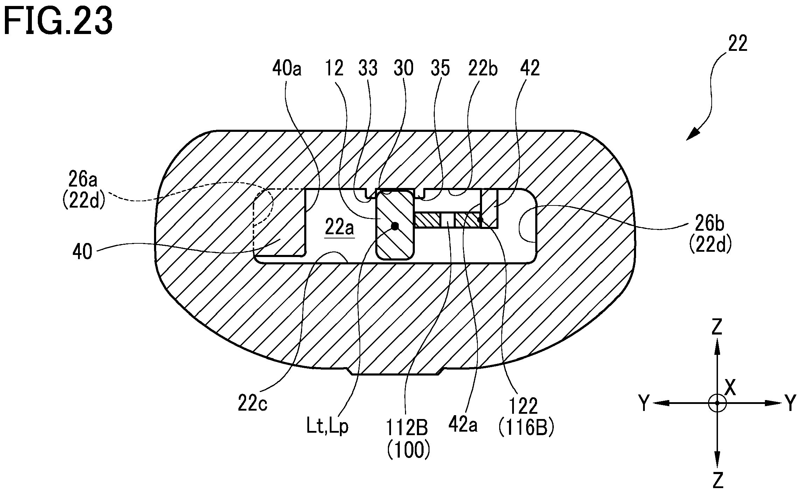

Subsequently, when the IOL 100 is further pushed toward the tip end 20a of the injection part 20, the IOL 100 is pushed into the passage 20b from the passage 22a. Specifically, as shown in FIGs. 22 and 23, the outer circumferential part 110a of the optical part 110 comes away from the inside surface 40a of the first protrusion 40, and the front-side support part 112A and the optical part 110 are moved into the passage 20b of the injection part 20. Thus, the optical part 110 is free from pushing by the first protrusion 40, and thereby moves toward the first inner wall surface 24a (the first inner wall surface 26a). As a result, the optical axis L of the optical part 110 is moved in a position of the central axis Lt of the passage 20b (the passage 22a).

At that time, to a portion 122 in the root portion 116B of the rear-side support part 112B on the second inner wall surface 26b side, the inside surface 42a of the second protrusion 42 on the second inner wall surface 26b side comes to contact. A posture (position) of the IOL 100 is thereby maintained.

The detailed explanation is given below. Firstly, the optical part 110 gets free from pushing by the first protrusion 40 and comes to contact with the inner wall surfaces 20c of the injection part 20 to be subjected to the stress by the inner wall surfaces 20c. Therefore, the optical part 110 is intended to rotate (about the optical axis L, for example) in a direction which the root portion 116B of the rear-side support part 112B comes close to the second inner wall surface 26b (in a clockwise direction in FIG. 22). However, the inside surface 42a of the second protrusion 42 is in contact with the root portion 116B of the rear-side support part 112B, and therefore the optical part 110 is restrained from rotation in the direction which the root portion 116B of the rear-side support part 112B comes close to the second inner wall surface 26b. As a result, the clearance C0 is formed and maintained between the root portion 116A of the front-side support part 112A and the first inner wall surface 26a of the setting part 22, and therefore a clearance C1 is also formed between the root portion 116A of the front-side support part 112A and the first inner wall surface 24a of the injection part 20. Accordingly, the root portion 116A of the front-side support part 112A is not interfered by the first inner wall surface 24a.

As mentioned above, in the present embodiment, the optical part 110 is pushed by the first protrusion 40, and hence the clearance C0 is formed between the root portion 116A of the front-side support part 112A and the first inner wall surface 26a. As a consequence, when the IOL 100 is subsequently pushed toward the tip end 20a in the passage 20b of the injection part 20, the clearance C1 becomes easy to be formed between the root portion 116A of the front-side support part 112A and the first inner wall surface 24a.

Further, the second protrusion 42 restrains the optical part 110 from rotation in the direction which the root portion 116B of the rear-side support part 112B comes close to the second inner wall surface 26b, and thus the clearance C1 becomes easy to be formed between the root portion 116A of the front-side support part 112A and the first inner wall surface 24a of the injection part 20.

Further, in the present embodiment, the first protrusion 40 pushes the optical part 110, and the second protrusion 42 restrains the optical part 110 from rotation in the direction which the root portion 116B of the rear-side support part 112B comes close to the second inner wall surface 26b. Therefore, even after the optical part 110 is separated from the first protrusion 40, the posture of the IOL 100 is unchanged from the state when the optical part 110 was in contact with the first protrusion 40. Accordingly, the clearance C1 becomes easy to be formed between the root portion 116A of the front-side support part 112A and the first inner wall surface 24a of the injection part 20.

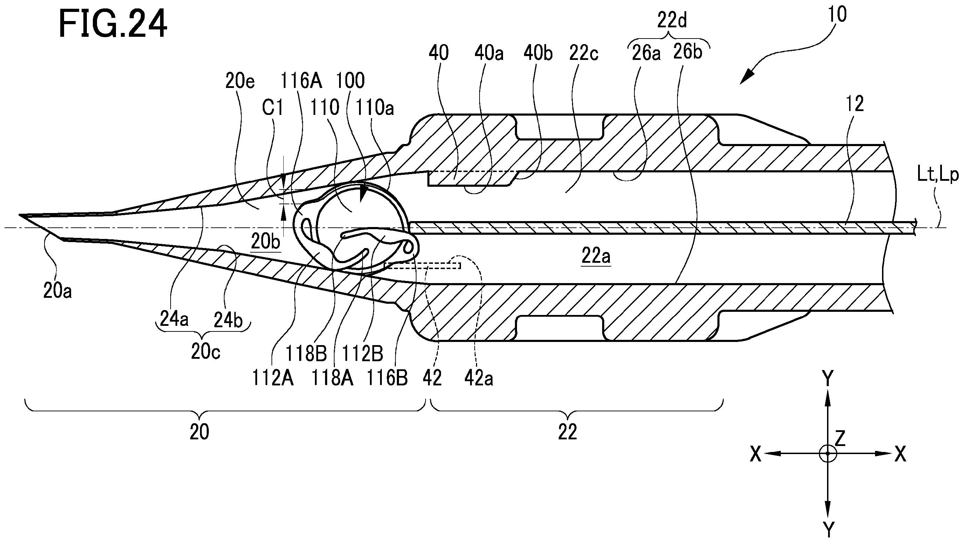

After that, when the IOL 100 is further pushed toward the tip end 20a of the injection part 20, as shown in FIG. 24, in the passage 20b, the outer circumferential part 110a of the optical part 110 is brought into contact with the inner wall surfaces 20c of the injection part 20, and the optical part 110 is folded as valley-folded toward the bottom surface 20e (toward a rear side of the paper in FIG. 24).

At the same time, the front-side support part 112A comes to contact with the second inner wall surface 24b, and thereby the root portion 116A is gradually curved and folded toward the optical part 110.

Herein, in the present embodiment, as shown in the above mentioned FIG. 22, the clearance C1 is formed between the root portion 116A of the front-side support part 112A and the first inner wall surface 24a. Therefore, during the push-out operation of the IOL 100, the root portion 116A of the front-side support part 112A is not interfered by the first inner wall surface 24a. While the root portion 116A of the front-side support part 112A is not interfered by the first inner wall surface 24a, the distal end portion 118A of the front-side support part 112A is firmly pressed by the second inner wall surface 24b. In short, the distal end portion 118A of the front-side support part 112A is easily subjected to the stress by the second inner wall surface 24b. Accordingly, the distal end portion 118A of the front-side support part 112A is guided to the inside of the optical part 110, and thus an entering amount of the front-side support part 112A getting in the inside of the optical part 110 is increased as shown in FIG. 24. This increase in the entering amount of the front-side support part 112A getting in the inside of the optical part 110 contributes to subsequently maintaining the front-side support part 112A to be placed on the surface of the optical part 110 on the ceiling surface 20d side.

In this manner, the distal end portion 118A of the front-side support part 112A is made to be placed inside the outer circumferential part 110a of the optical part 110 so that the front-side support part 112A gets in the inside of the optical part 110 (more inward than the outer circumferential part 110a). In other words, the distal end portion 118A of the front-side support part 112A enters into a space between the optical part 110 and the ceiling surface 20d.

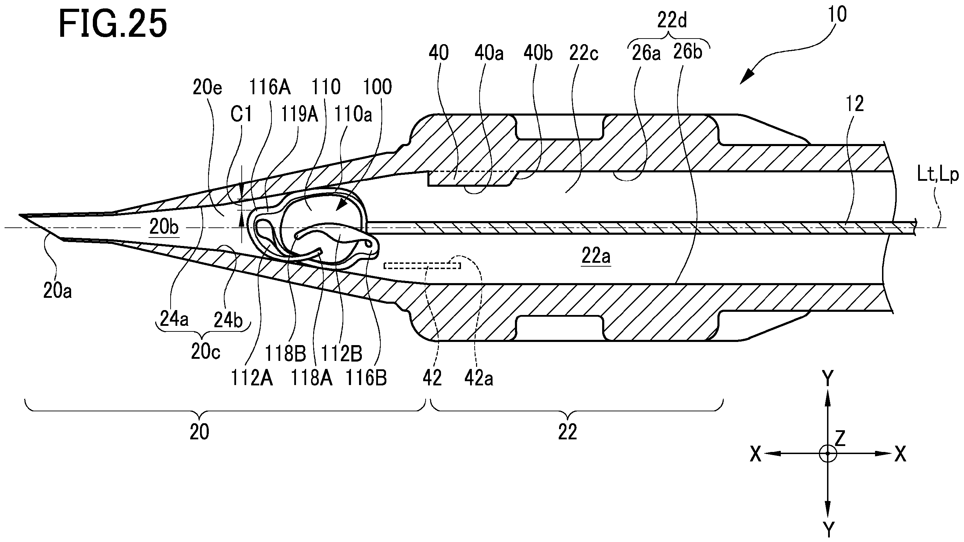

After that, the IOL 100 is further pushed toward the tip end 20a of the injection part 20, and then the optical part 110 is further folded as valley-folded as shown in FIG. 25.

At this time, if the clearance C1 is not formed between the root portion 116A of the front-side support part 112A and the first inner wall surface 24a, the root portion 116A of the front-side support part 112A has a possibility of being folded and inwardly twisted in the passage 20b in conformity with a shape of the first inner wall surface 24a. In such a case, when the IOL 100 is further pushed toward the tip end 20a of the injection part 20, the front-side support part 112A having an elastic force attempts to extend to relieve the twisted state. As a result, there is a possibility that the front-side support part 112A comes out of the optical part 110.

On the contrary, in the present embodiment, the clearance C1 is formed between the root portion 116A of the front-side support part 112A and the first inner wall surface 24a, and the root portion 116A of the front-side support part 112A is hence separated from the first inner wall surface 24a. Therefore, the root portion 116A of the front-side support part 112A is hardly twisted and folded inwardly in the passage 20b in conformity with the shape of the first inner wall surface 24a. Further, the root portion 116A of the front-side support part 112A is in a rising position (an outer circumferential surface 119A of the root portion 116A is placed on a front side of the paper in FIG. 25) following valley-folding of the optical part 110. This rising position of the root portion 116A of the front-side support part 112A makes it easy to subsequently maintain a state in which the front-side support part 112A is placed on the surface of the optical part 110 on the ceiling surface 20d side.

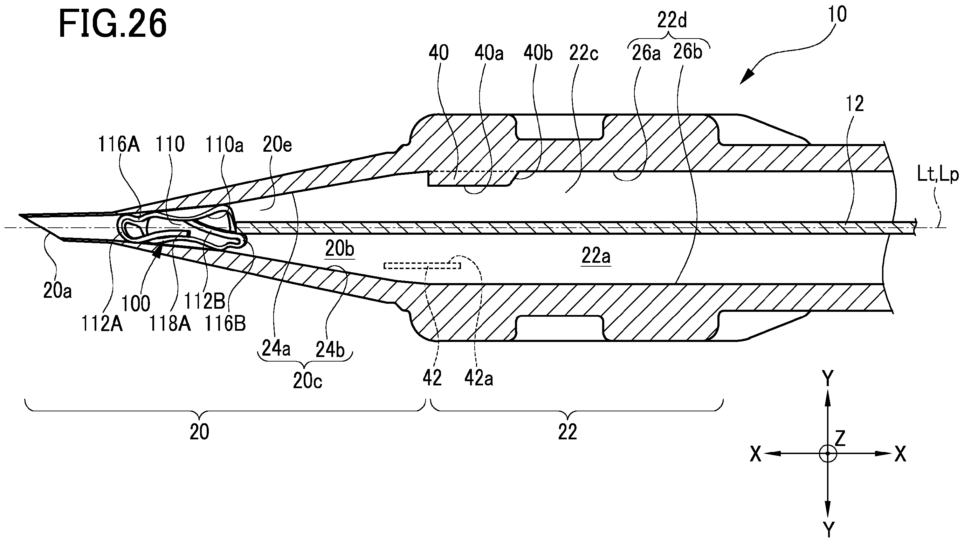

After that, the IOL 100 is further pushed toward the tip end 20a of the injection part 20, and as shown in FIG. 26, the root portion 116A is further curved to fold the front-side support part 112A, and the front-side support part 112A is kept to be placed on top of the optical part 110 on the ceiling surface 20d side. Further, the optical part 110 is further valley-folded toward the rear side of the paper in FIG. 26 to be folded into a tiny piece. Incidentally, the rear-side support part 112B is also kept to be placed on top of the optical part 110 on the ceiling surface 20d side.

In the present embodiment, the entering amount of the front-side support part 112A getting in the inside of the optical part 110 is increased as shown in the above mentioned FIG. 24, and the root portion 116A of the front-side support part 112A is in the rising position as shown in the above mentioned FIG. 25. Accordingly, even if the IOL 100 being pushed in the passage 20b is oscillated, it is enabled to maintain the state in which the front-side support part 112A is placed on top of the optical part 110 on the ceiling surface 20d side.

Further, after that, when the IOL 100 is further pushed toward the tip end 20a of the injection part 20, while the front-side support part 112A and the rear-side support part 112B are placed on top of the optical part 110 on the ceiling surface 20d side and the optical part 110 is valley-folded toward the bottom surface 20e into a tiny piece, the IOL 100 is pushed out of the injection part 20 through the tip end 20a of the injection part 20 and moved into the eye. In this manner, the folded IOL 100 is injected into the eye.

The above mentioned embodiment illustrates an example that after the outer circumferential part 110a of the optical part 110 moves away from the inside surface 40a of the first protrusion 40, the root portion 116B of the rear-side support part 112B comes to contact with the inside surface 42a of the second protrusion 42. As one alternative, the root portion 116B of the rear-side support part 112B may be configured to come to contact with the inside surface 42a of the second protrusion 42 simultaneously with the time when the outer circumferential part 110a of the optical part 110 moves away from the inside surface 40a of the first protrusion 40.

Further, the first protrusion 40 may be formed separately from the first inner wall surface 26a in a position between the first inner wall surface 26a and the central axis Lt of the passage 22a. As another alternative, the second protrusion 42 may be formed to protrude from the second inner wall surface 26b toward the central axis Lt of the passage 22a.

As mentioned above, according to the present disclosure, in the setting part 22, the inside surface 40a of the first protrusion 40 is formed in a position closer to the central axis Lt of the passage 22a than the first inner wall surface 26a. Further, the inside surface 40a of the first protrusion 40 is formed to be brought into contact with the outer circumferential part 110a of the optical part 110 during the push-out operation of the IOL 100.

Therefore, the optical part 110 is pushed by the first protrusion 40 and thus moved toward the central axis Lt of the passage 22a from the first inner wall surface 26a side. Thus, the clearance C0 is formed between the root portion 116A of the front-side support part 112A and the first inner wall surface 26a. Accordingly, after that, when the IOL 100 is pushed toward the tip end 20a in the passage 20b of the injection part 20, the clearance C1 is easily formed between the root portion 116A of the front-side support part 112A and the first inner wall surface 24a. This clearance C1 therefore prevents the root portion 116A of the front-side support part 112A from being interfered by the first inner wall surface 24a.

Further, the root portion 116A of the front-side support part 112A is thus hard to be interfered by the first inner wall surface 24a, and on the other hand, the distal end portion 118A of the front-side support part 112A is firmly pressed against the second inner wall surface 24b. Therefore, the distal end portion 118A of the front-side support part 112A is easily subjected to the stress by the second inner wall surface 24b. The distal end portion 118A of the front-side support part 112A is hence guided to the inside of the optical part 110, and thus the entering amount of the front-side support part 112A getting in the inside of the optical part 110 is increased. This increased entering amount of the front-side support part 112A getting in the inside of the optical part 110 contributes to subsequently maintaining the state in which the front-side support part 112A is placed on top of the optical part 110 on the ceiling surface 20d side. Accordingly, the IOL 100 can be folded into a tiny piece with the front-side support part 112A (and the rear-side support part 112B) being folded and placed on the optical part 110. As a result, the IOL 100 can be appropriately deformed and injected.

Further, the distance D between the inside surface 40a of the first protrusion 40 and the central axis Lt of the passage 22a is shorter than the radius of the optical part 110. Therefore, when the outer circumferential part 110a of the optical part 110 comes to contact with the inside surface 40a of the first protrusion 40, the optical axis L of the optical part 110 comes off the central axis Lt of the passage 22a and moves closer to the second inner wall surface 26b than the central axis Lt. As a result, the clearance C0 is formed between the root portion 116A of the front-side support part 112A and the first inner wall surface 26a.

Further, the inside surface 40a of the first protrusion 40 is formed in a peripheral end portion of the first protrusion 40 protruding toward the central axis Lt of the passage 22a from the first inner wall surface 26a. The optical part 110 is pushed by the first protrusion 40, and thus the clearance C0 is formed between the root portion 116A of the front-side support part 112A and the first inner wall surface 26a. Accordingly, after that, when the front-side support part 112A is placed in the passage 20b, the clearance C1 becomes easy to be formed between the root portion 116A of the front-side support part 112A and the first inner wall surface 24a.

Further, in the setting part 22, the inside surface 42a of the second protrusion 42 is formed on a side closer to the central axis Lt of the passage 22a than the second inner wall surface 26b. The inside surface 42a of the second protrusion 42 is positioned to be brought into contact with the root portion 116B of the rear-side support part 112B on the second inner wall surface 26b side when or after the outer circumferential part 110a of the optical part 110 is separated from the inside surface 40a of the first protrusion 40 during the push-out operation of the IOL 100.

Thus, even after the optical part 110 is separated from the first protrusion 40, the posture of the IOL 100 (a circumferential direction of the optical part 110) is unchanged from the posture when the optical part 110 was in contact with the first protrusion 40. As a result, the clearance C1 is formed between the root portion 116A of the front-side support part 112A and the first inner wall surface 24a of the injection part 20. Therefore, the root portion 116A of the front-side support part 112A is not interfered by the first inner wall surface 24a, and thus the front-side support part 112A becomes easily folded inside the optical part 110. Accordingly, the front-side support part 112A becomes easily placed on the surface of the optical part 110 on the ceiling surface 20d side. Herein, "when or after the optical part 110 is separated from the inside surface 40a of the first protrusion 40" means the simultaneous time with the timing when the optical part 110 moves away from the inside surface 40a of the first protrusion 40 or the time after the optical part 110 moves away from the inside surface 40a of the first protrusion 40.

Further, the inside surface 42a of the second protrusion 42 is formed to be brought into contact with the root portion 116B of the rear-side support part 112B when the front-side support part 112A and the optical part 110 are placed in the passage 20b of the injection part 20 during the push-out operation of the IOL 100. Thus, even if the optical part 110 comes to contact with the inner wall surfaces 20c of the injection part 20 and is subjected to the stress by the surfaces 20c, the posture of the IOL 100 is unchanged from the state when the optical part 110 was in contact with the first protrusion 40.

### (Third Embodiment)

As a third embodiment, a configuration is given by combining the above mentioned first and second embodiments. As one example of such a configuration, the injector 1 of the present embodiment includes the protruding portion 36, the first protrusion 40, and the second protrusion 42.

According to the present embodiment, during the push-out operation of the IOL 100 by the plunger 12, the optical part 110 is pushed by the first protrusion 40, and hence the clearance C0 is formed between the root portion 116A of the front-side support part 112A and the first inner wall surface 26a. Subsequently, when the IOL 100 is pushed toward the tip end 20a in the passage 20b of the injection part 20, the posture of the IOL 100 is maintained by the second protrusion 42, and thus the clearance C1 is formed between the root portion 116A of the front-side support part 112A and the first inner wall surface 24a. Therefore, the root portion 116A of the front-side support part 112A is hard to be interfered by the first inner wall surface 24a.

After the root portion 116A of the front-side support part 112A is thus made hard to be interfered by the first inner wall surface 24a, the leading end 12a of the plunger 12 is directed toward the first inner wall surface 24a or the second inner wall surface 24b by the protruding portion 36, so that the orientation of the IOL 100 in the circumferential direction of the optical part 110 is easily controlled. Accordingly, the IOL 100 is easily rotated in the circumferential direction of the optical part 110 such that the root portion 116A of the front-side support part 112A moves away from the first inner wall surface 24a and comes close to the push-out axis Lp. Therefore, the entering amount δ1 of the front-side support part 112A can be controlled to be large, and hence the front-side support part 112A is kept to be folded and placed on top of the optical part 110 when the IOL 100 is about to be injected into the eye. Accordingly, the IOL 100 can be appropriately deformed and injected.

An embodiment combining the first and second embodiments is not limited to the above third embodiment. At least any one of configurations included in the first embodiment and any one of configurations included in the second embodiment may be combined. For example, the injector 1 may only include the protruding portion 36 and the first protrusion 40. Namely, the injector 1 configured by combining the first and second embodiments may not include the second protrusion 42. In other words, the injector 1 may be embodied in such a way that the optical part 110 is pressed by the first protrusion 40, and the protruding portion 36 directs the leading end 12a of the plunger 12 toward first inner wall surface 24a or the second inner wall surface 24b.

### Reference Signs List

- 1: Injector
- 10: Cylinder body (Body)
- 12: Plunger
- 12a: Leading end
- 20: Injection part
- 20a: Tip end
- 20b: Passage
- 20c: Inner wall surfaces
- 20d: Ceiling surface
- 20e: Bottom surface
- 22: Setting part
- 22a: Passage
- 22b: Ceiling surface
- 22c: Bottom surface
- 22d: Inner wall surfaces
- 24a: First inner wall surface
- 24b: Second inner wall surface
- 26a: First inner wall surface
- 26b: Second inner wall surface
- 30: Guide portion
- 32: First protrusion
- 34: Second protrusion
- 36: Protruding portion
- 36a: Guide face
- 40: First protrusion
- 40a: Inside surface
- 42: Second protrusion
- 42a: Inside surface
- 100: Intraocular lens (IOL)
- 110: Optical part
- 110a: Outer circumferential part
- 112A: Front-side support part
- 112B: Rear-side support part
- 116A: Root portion
- 116B: Root portion
- 118A: Distal end portion
- 118B: Distal end portion
- 120: End portion
- 122: Portion (in the root portion of the rear-side support part)
- Lt: Central axis (of the passage)
- Lp: Push-out axis
- C0: Clearance
- C1: Clearance
- D: Distance

## Claims

1. An intraocular lens injection instrument (1) for injecting an intraocular lens (100) into an eye, the intraocular lens (100) comprising an optical part (110) of a disk-like shape and a first support part (112A) extending outwardly from an outer circumferential part (110a) of the optical part (110a), wherein
the intraocular lens injection instrument (1) comprises:
a passage (20b) having a passage area gradually decreasing toward a tip end (20a) of the intraocular lens injection instrument (1);
inner wall surfaces (20c) formed on both sides in a direction orthogonal to an axial direction of the passage (20b), the direction being parallel to a radial direction of the optical part (110) when the intraocular lens (100) is placed in the passage (20b);
a ceiling surface (20d) and a bottom surface (20e) formed, respectively, on each of both sides in a direction orthogonal to the axial direction of the passage (20b), the direction being parallel to an axial direction (L) of the optical part (110) when the intraocular lens (100) is placed in the passage (20b),
a push member (12) configured to push out the intraocular lens (100), the push member including a contact portion (12a) to be placed in contact with the intraocular lens (100); and
a lens control mechanism to control an orientation of the intraocular lens (100) in a circumferential direction of the optical part (110), **characterized in that**
the intraocular lens injection instrument (1) further comprises
protrusions (32, 34) protruding from the ceiling surface (20d) toward the bottom surface(20e) and constituting wall surfaces of a guide portion (30) provided on the ceiling surface (20d) to guide a push-out direction (Lp) of the push member (12), and
the lens control mechanism (36) is formed on an end side of the protrusion (32) close to the tip end (20a) to protrude toward an inside of the guide portion (30),
the lens control mechanism (36) protrudes from the ceiling surface (20d) toward the bottom surface(20e) in the passage (20b),
the lens control mechanism being configured to direct movement of the contact portion (12a) of the push member (12) toward one of the inner wall surfaces (20c) when or after the intraocular lens (100) with the first support part (112A) positioned ahead of the optical part (110) toward the tip end (20a) in the passage (20b) is pushed toward the tip end (20a) by the push member (12) and then the first support part (112A) is folded so that a distal end portion (118A) of the first support part (112A) is disposed more inward than the outer circumferential part (110a) of the optical part (110).

2. The intraocular lens injection instrument (1) according to claim 1, **characterized in that**
the intraocular lens (100) further comprises a second support part (112B) extending outwardly from the outer circumferential part (110a) of the optical part (110),
the first support part (112A) and the second support part (112B) are formed in point-symmetrical positions with reference to a center of the optical part (110),
the inner wall surfaces (20c) include a first inner wall surface (24a) and a second inner wall surface (24b) formed on each of the both sides of the passage, respectively,
a root portion (116A) of the first support part (112A) is placed in a position closer to the first inner wall surface (24a) than a central axis (Lt) of the passage (20b),
a root portion (116B) of the second support part (112B) is placed in a position closer to the second inner wall surface (24b) than the central axis (Lt) of the passage (20b), and
the lens control mechanism is configured to direct movement of the contact portion (12a) of the push member (12) toward the first inner wall surface (24a).

3. The intraocular lens injection instrument (1) according to claim 1 or 2, **characterized in that**
the contact portion (12a) of the push member (12) is moved forward along a push axis (Lp),
the lens control mechanism is an inclined passage constituting at least a part of the passage (20b), and
a central axis (Lt) of the inclined passage (20b) is inclined with respect to the push-out axis (Lp) to a radial direction of the optical part (110) when the intraocular lens (100) is placed in the passage (20b).

4. The intraocular lens injection instrument (1) according to any one of claims 1 to 3, suitable for pushing the intraocular lens (100) with the first support part (112A) positioned ahead of the optical part (110) toward the tip end (20a) in the rear-side passage (22a) toward the passage (20b) by the push member (12) and then pushing the intraocular lens (100) toward the tip end (20a) by the push member (12) in the passage (20b) so that the first support part (112A) is folded inside the optical part (110) during injection of the intraocular lens (100) into the eye,
**characterized in that** the intraocular lens injection instrument (1) further comprises:
a rear-side passage (22a) formed on a rear side of the passage (20b) in a push-out direction (Lp) of the intraocular lens (100); and
a first rear-side inner wall surface (26a) and a second rear-side inner wall surface (26b) formed, respectively, on each of both sides in a direction orthogonal to an axial direction of the rear-side passage (22a), the direction being parallel to a radial direction of the optical part (110) when the intraocular lens (100) is placed in the rear-side passage (22a),
the first rear-side inner wall surface (26a) is provided on a side where a root portion (116A) of the first support part (112A) is placed in the rear-side passage (20b) with respect to the central axis (Lt) of the rear-side passage (20b) when the intraocular lens (100) is placed therein,
a first contact portion (40a) is provided on an end of a first protrusion (40) which protrudes from the first rear side inner wall surface (26a) toward the central axis (Lt) of the rear side passage (20b) in a position closer to the central axis (Lt) of the rear-side passage (22a) than the first rear-side inner wall surface (26a), and
the first contact portion (40a) is formed to be in contact with the outer circumferential part (110a) of the optical part (110) when the intraocular lens (100) is pushed out.

## Patentansprüche

1. Ein Intraokularlinsen-Injektionsinstrument (1) zum Injizieren einer Intraokularlinse (100) in ein Auge, wobei die Intraokularlinse (100) einen optischen Abschnitt (110) mit einer scheibenartigen Form und einen ersten Stützabschnitt (112A), der sich von einem äußeren Umfangsabschnitt (110) des optischen Abschnitts (110a) nach außen erstreckt, aufweist, wobei
das Intraokularlinsen-Injektionsinstrument (1) umfasst:
einen Durchlass (20b), der eine zum Spitzenende (20a) des Intraokularlinsen-Injektionsinstruments (1) graduell abnehmende Durchlassfläche aufweist;
Innenwandflächen (20c), die auf beiden Seiten in einer Richtung senkrecht zu einer axialen Richtung des Durchlasses (20b) ausgebildet sind, wobei die Richtung parallel zu einer radialen Richtung des optischen Abschnitts (110) ist, wenn die Intraokularlinse (199) im Durchlass (20b) platziert ist;
eine Deckenfläche (20d) und eine Bodenfläche (20e), die jeweils auf jeder der beiden Seiten in einer Richtung senkrecht zur axialen Richtung des Durchlasses (20b) ausgebildet sind, wobei die Richtung parallel zu einer axialen Richtung (L) des optischen Abschnitts (110) ist, wenn die Intraokularlinse (100) im Durchlass (20b) platziert ist;
ein Druckelement (12), das dazu ausgebildet ist, die Intraokularlinse (100) herauszudrücken, wobei das Druckelement einen Kontaktabschnitt umfasst, um in Kontakt mit der Intraokularlinse (100) platziert zu werden; und
einen Linsensteuermechanismus, um eine Orientierung der Intraokularlinse (100) in einer Umfangsrichtung des optischen Abschnitts (110) zu steuern, **dadurch gekennzeichnet, dass**
das Intraokularlinsen-Injektionsinstrument (1) weiter umfasst:
Vorsprünge (32, 34), die von der Deckenfläche (20d) in Richtung der Bodenfläche (20e) vorstehen und Wandflächen eines Führungsabschnitts (30) bilden, der an der Deckenfläche (20e) vorgesehen ist, um eine Herausdrückrichtung (Lp) des Druckelements (12) zu führen, und
der Linsensteuermechanismus (36) an einer Endseite des Vorsprungs (32) nahe dem Spitzenende (20a) ausgebildet ist, um in Richtung einer Innenseite des Führungsabschnitts (30) vorzustehen,
wobei der Linsensteuerungsmechanismus (36) von der Deckenfläche (20d) in Richtung der Bodenfläche (20e) im Durchlass (20b) vorsteht,
wobei der Linsensteuerungsmechanismus dazu ausgebildet ist, die Bewegung des Kontaktabschnitts (12a) des Druckelements (12) in Richtung einer der Innenwandflächen (20c) zu lenken, wenn oder nachdem die Intraokularlinse (100) mit dem ersten Stützabschnitt (112A), der vor dem optischen Abschnitt (110) in Richtung des Spitzenendes (20a) im Durchlass (20b) angeordnet ist, in Richtung des Spitzenendes (20a) durch das Druckelement (12) gedrückt wird, und anschließend der erste Stützabschnitt (112A) gefaltet wird, so dass ein distaler Endabschnitt (118A) des ersten Stützabschnitts (112A) weiter innen angeordnet ist als der äußere Umfangsabschnitt (110a) des optischen Abschnitts (110).

2. Das Intraokularlinsen-Injektionsinstrument (1) nach Anspruch 1, **dadurch gekennzeichnet, dass**
die Intraokularlinse (100) ferner einen zweiten Stützabschnitt (112B) umfasst, der sich vom äußeren Umfangsabschnitt (110a) des optischen Abschnitts (110) nach außen erstreckt,
der erste Stützabschnitt (112A) und der zweite Stützabschnitt (112B) in punktsymmetrischen Positionen in Bezug auf eine Mitte des optischen Abschnitts (110) ausgebildet sind,
die Innenwandflächen (20c) eine erste Innenwandfläche (24a) und eine zweite Innenwandfläche (24b), die jeweils auf jeder der beiden Seiten des Durchlasses ausgebildet sind, umfassen,
ein Fußpunktabschnitt (116A) des ersten Stützabschnitts (112A) in einer Position näher zur ersten Innenwandfläche (24a) platziert ist als die Mittelachse (Lt) des Durchlasses (20b)
ein Fußpunktabschnitt (116B) des zweiten Stützabschnitts (112B) in einer Position näher zur zweiten Innenwandfläche (24b) platziert ist als die Mittelachse (Lt) des Durchlasses (20b) und
der Linsensteuermechanismus dazu ausgebildet ist, die Bewegung des Kontaktabschnitts (12a) des Druckelements (12) in Richtung der ersten Innenwandfläche (24a) zu lenken.

3. Das Intraokularlinsen-Injektionsinstrument (1) nach Anspruch 1 oder 2, **dadurch gekennzeichnet, dass**
der Kontaktabschnitt (12a) des Druckelements (12) entlang einer Druckachse (Lp) vorwärts bewegt wird,
der Linsensteuermechanismus ein geneigter Durchlass ist, der zumindest einen Abschnitt des Durchlasses (20b) bildet, und
sich eine Mittelachse (Lt) des geneigten Durchlasses (20b) in Bezug auf die Herausdrückachse (Lp) zu einer radialen Richtung des optischen Abschnitts (110) neigt, wenn die Intraokularlinse (100) im Durchlass (20b) platziert ist.

4. Das Intraokularlinsen-Injektionsinstrument (1) nach einem der Ansprüche 1 bis 3, das dazu geeignet ist, die Intraokularlinse (100) mit dem ersten Stützabschnitt (112A), der vor dem optischen Abschnitt (110) in Richtung des Spitzenendes (20a) im rückseitigen Durchlass (22a) angeordnet ist, in Richtung des Durchlasses (20b) durch das Druckelement (12) zu drücken und anschließend die Intraokularlinse (100) in Richtung des Spitzenendes (20a) durch das Druckelement (12) im Durchlass (20b) zu drücken, so dass der erste Stützabschnitt (112A) im optischen Abschnitt (110) während der Injektion der Intraokularlinse ins Auge gefaltet wird,
**dadurch gekennzeichnet, dass** das Intraokularlinsen-Injektionsinstrument (1) ferner umfasst:
einen rückseitigen Durchlass (22a), der auf einer rückwärtigen Seite des Durchlasses (20b) in einer Herausdrückrichtung (Lp) der Intraokularlinse (100) ausgebildet ist; und
einer erste rückseitige Innenwandfläche (26a) und eine zweite rückseitige Innenwandfläche (26b), die jeweils auf jeder der beiden Seiten in einer Richtung senkrecht zu einer axialen Richtung des rückseitigen Durchlasses (22a) ausgebildet sind, wobei die Richtung parallel zu einer radialen Richtung des optischen Abschnitts ist, wenn die Intraokularlinse (100) im rückseitigen Durchlass (22a) platziert ist,
wobei die erste rückseitige Innenwandfläche (26a) auf einer Seite vorgesehen, auf der ein Fußpunktabschnitt (116A) des ersten Stützabschnitts (112A) im rückseitigen Durchlass (20b) in Bezug auf die Mittelachse (Lt) des rückseitigen Durchlasses (20b) platziert ist, wenn die Intraokularlinse (100) darin platziert ist,
wobei ein erster Kontaktabschnitt (40a) an einem Ende eines ersten Vorsprungs (40) vorgesehen ist, der von der ersten rückseitigen Innenwandfläche (26a) in Richtung der Mittelachse (Lt) des rückseitigen Durchlasses (20b) in einer Position näher zur Mittelachse (Lt) des rückseitigen Durchlasses (22a) als die erste rückseitige Innenwandfläche (26a) vorsteht, und
wobei der erste Kontaktabschnitt (40a) ausgebildet ist, um mit dem äußeren Umfangsabschnitt (110a) des optischen Abschnitts (110) in Kontakt zu sein, wenn die Intraokularlinse (100) herausgedrückt wird.

## Revendications

1. Instrument d'injection de lentille intraoculaire (1) pour injecter une lentille intraoculaire (100) dans un œil, la lentille intraoculaire (100) comprenant une partie optique (110) en forme de disque et une première partie de support (112A) s'étendant vers l'extérieur à partir d'une partie circonférentielle extérieure (110a) de la partie optique (110a), dans lequel
l'instrument d'injection de lentille intraoculaire (1) comprend :
un passage (20b) ayant une zone de passage diminuant graduellement en direction d'une extrémité de pointe (20a) de l'instrument d'injection de lentille intraoculaire (1) ;
des surfaces de paroi intérieures (20c) formées sur les deux côtés dans une direction orthogonale à une direction axiale du passage (20b), la direction étant parallèle à une direction radiale de la partie optique (110) lorsque la lentille intraoculaire (100) est placée dans le passage (20b) ;
une surface de plafond (20d) et une surface inférieure (20e) formées, respectivement, sur chacun des deux côtés dans une direction orthogonale à la direction axiale du passage (20b), la direction étant parallèle à une direction axiale (L) de la partie optique (110) lorsque la lentille intraoculaire (100) est placée dans le passage (20b),
un élément poussoir (12) configuré pour expulser la lentille intraoculaire (100), l'élément poussoir incluant une partie de contact (12a) à placer en contact avec la lentille intraoculaire (100) ; et
un mécanisme de commande de lentille pour commander une orientation de la lentille intraoculaire (100) dans une direction circonférentielle de la partie optique (110), **caractérisé en ce que**
l'instrument d'injection de lentille intraoculaire (1) comprend en outre
des saillies (32, 34) faisant saillie de la surface de plafond (20d) en direction de la surface inférieure (20e) et constituant des surfaces de paroi d'une partie de guidage (30) fournie sur la surface de plafond (20d) pour guider une direction d'expulsion (Lp) de l'élément poussoir (12), et
le mécanisme de commande de lentille (36) est formé sur un côté d'extrémité de la saillie (32) à proximité de l'extrémité de pointe (20a) pour faire saillie en direction d'un intérieur de la partie de guidage (30),
le mécanisme de commande de lentille (36) fait saillie de la surface de plafond (20d) en direction de la surface inférieure (20e) dans le passage (20b),
le mécanisme de commande de lentille étant configuré pour diriger le mouvement de la partie de contact (12a) de l'élément poussoir (12) en direction de l'une des surfaces de paroi intérieures (20c) lorsque ou après que la lentille intraoculaire (100) avec la première partie de support (112A) positionnée devant la partie optique (110) en direction de l'extrémité de pointe (20a) dans le passage (20b) est poussée en direction de l'extrémité de pointe (20a) par l'élément poussoir (12) puis la première partie de support (112A) est pliée de manière à ce qu'une partie extrémité distale (118A) de la première partie de support (112A) soit disposée plus vers l'intérieur que la partie circonférentielle extérieure (110a) de la partie optique (110).

2. Instrument d'injection de lentille intraoculaire (1) selon la revendication 1, **caractérisé en ce que**
la lentille intraoculaire (100) comprend en outre une seconde partie de support (112B) s'étendant vers l'extérieur à partir de la partie circonférentielle extérieure (110a) de la partie optique (110),
la première partie de support (112A) et la seconde partie de support (112B) sont formées à des positions centralement symétriques par référence à un centre de la partie optique (110),
les surfaces de paroi intérieures (20c) comprennent une première surface de paroi intérieure (24a) et une seconde surface de paroi intérieure (24b) formées sur chacun des deux côtés du passage, respectivement,
une partie base (116A) de la première partie de support (112A) est placée dans une position plus proche de la première surface de paroi intérieure (24a) qu'un axe central (Lt) du passage (20b),
une partie base (116B) de la seconde partie de support (112B) est placée dans une position plus proche de la seconde surface de paroi intérieure (24b) que l'axe central (Lt) du passage (20b), et
le mécanisme de commande de lentille est configuré pour diriger le mouvement de la partie de contact (12a) de l'élément poussoir (12) en direction de la première surface de paroi intérieure (24a).

3. Instrument d'injection de lentille intraoculaire (1) selon la revendication 1 ou 2, **caractérisé en ce que**
la partie de contact (12a) de l'élément poussoir (12) est avancée le long d'un axe d'expulsion (Lp),
le mécanisme de commande de lentille est un passage incliné constituant au moins une partie du passage (20b), et
un axe central (Lt) du passage incliné (20b) est incliné par rapport à l'axe d'expulsion (Lp) vers une direction radiale de la partie optique (110) lorsque la lentille intraoculaire (100) est placée dans le passage (20b).

4. Instrument d'injection de lentille intraoculaire (1) selon l'une quelconque des revendications 1 à 3, adapté à pousser la lentille intraoculaire (100) avec la première partie de support (112A) positionnée devant la partie optique (110) en direction de l'extrémité de pointe (20a) dans le passage latéral arrière (22a) en direction du passage (20b) par l'élément poussoir (12) puis à pousser la lentille intraoculaire (100) en direction de l'extrémité de pointe (20a) par l'élément poussoir (12) dans le passage (20b) de manière à ce que la première partie de support (112A) soit pliée à l'intérieur de la partie optique (110) pendant l'injection de la lentille intraoculaire (100) dans l'œil,
**caractérisé en ce que** l'instrument d'injection de lentille intraoculaire (1) comprend en outre :
un passage latéral arrière (22a) formé sur un côté arrière du passage (20b) dans une direction d'expulsion (Lp) de la lentille intraoculaire (100) ; et
une première surface de paroi intérieure latérale arrière (26a) et une seconde surface de paroi intérieure latérale arrière (26b) formée, respectivement, sur chacun des deux côtés dans une direction orthogonale à une direction axiale du passage latéral arrière (22a), la direction étant parallèle à une direction radiale de la partie optique (110) lorsque la lentille intraoculaire (100) est placée dans le passage latéral arrière (22a),
la première surface de paroi intérieure latérale arrière (26a) est fournie sur un côté où une partie base (116A) de la première partie de support (112A) est placée dans le passage latéral arrière (20b) par rapport à l'axe central (Lt) du passage latéral arrière (20b) lorsque la lentille intraoculaire (100) est placée à l'intérieur de celui-ci,
une première partie de contact (40a) est fournie sur une extrémité d'une première saillie (40) qui fait saillie de la première surface de paroi intérieure latérale arrière (26a) en direction de l'axe central (Lt) du passage latéral arrière (20b) dans une position plus proche de l'axe central (Lt) du passage latéral arrière (22a) que la première surface de paroi latérale arrière (26a), et
la première partie de contact (40a) est formée pour être en contact avec la partie circonférentielle extérieure (110a) de la partie optique (110) lorsque la lentille intraoculaire (100) est expulsée.
